# EUROPEAN PATENT SPECIFICATION

(11) **EP 1 977 705 B1**
(45) Date of publication and mention of the grant of the patent: **15.02.2012**
(21) Application number: 08006291.2
(22) Date of filing: 31.03.2008
(51) Int. Cl.: A61B 17/34, A61B 18/14, A61B 1/05, A61B 1/07, A61B 17/32, A61B 19/00, A61B 1/313

(54) **Trocar**
Trokar
Trocart

(30) Priority: 02.04.2007 US 695146
(43) Date of publication of application: 08.10.2008
(73) Proprietor: Olympus Medical Systems Corp., Tokyo 151-0072 (JP)
(72) Inventor: Norikiyo, Shibata, Tokyo 192-8512 (JP); Sekino, Naomi, Tokyo 192-8512 (JP); Hideo, Sanai, Tokyo 192-8512 (JP)
(74) Representative: von Hellfeld, Axel

(56) References cited:
- EP-A- 0 624 346
- EP-A- 0 761 171
- US-A- 5 674 184

## Description

The present invention relates to a trocar used for forming an opening portion as an insertion hole for an instrument such as a rigid scope in an abdominal wall, for example, when the instrument is inserted into the abdominal wall of a patient.

Generally, when an instrument is inserted into an abdominal cavity in a laparoscopical operation, a cannula artificially provided in an abdominal wall is used as an insertion port. At this time, a trocar is used as a tool for forming the insertion port. The trocar has a cannula and an inner needle inserted into the cannula. At a use time of the trocar, the trocar assembled in a state that the inner needle is inserted into the cannula is punctured into an abdominal wall.

For example, U.S.PATENT APPLICATION PUBLICATION No. 5,334,150 specification (Patent Document 1) shows a trocar that is used while an aspect of puncturing an abdominal wall is being observed when a cannula is placed in the abdominal wall. In the trocar, a distal end of an inner needle has a transparent sharpened shape. The inner needle is formed with a hole portion into which an endoscope (a rigid scope) with a hard insertion portion can be inserted. Such a configuration is adopted that the insertion portion of the rigid scope is inserted into the hole portion from an opening provided in an end portion of the inner needle on a near side of an operator for use. Specifically, an image obtained from the rigid scope through a transparent distal end is converted to a video signal in a video processor. The video signal outputted from the video processor is inputted into a monitor and the image obtained from the rigid scope is displayed on the monitor. Thereby, puncture into the abdominal wall is performed while an aspect where the distal end of the inner needle advances into the abdominal wall is being observed.

U.S.PATENT APPLICATION PUBLICATION No. 5,569,292 specification (Patent Document 2) shows an obtuse trocar where a distal end of an inner needle is transparent and it is not sharpened. U.S.PATENT APPLICATION PUBLICATION No. 5,599,348 specification (Patent Document 3) shows a configuration of a trocar that facilitates puncture utilizing a high-frequency current to reduce an amount of puncture force. JPN.PAT.APPLN.KOKAI PUBLICATION No. 2002-85337 publication (Patent Document 4) discloses a configuration that transmits ultrasonic vibrations to a puncture portion to be punctured into a body wall and facilitates puncture of a trocar into the body wall utilizing ultrasonic waves.

With the trocar of Patent Document 1, the abdominal wall is punctured while being observed. Therefore, when the trocar is pushed through the abdominal wall, the saphenous arteries and the saphenous veins can be recognized beforehand. When the trocar is pushed through the abdominal wall, it may be possible to circumvent the saphenous arteries and the saphenous veins. However, the steep shape of the tip end of the inner needle of the trocar and the acute edges of the trigonal pyramid are likely to damage the blood vessels of the abdominal wall or the tissues of a muscle layer.

To prevent damage to the blood vessels of the abdominal wall or the tissues of a muscle layer, the trocar of Patent Document 2 has an inner needle whose tip end is not steep. In this case, the trocar can be pushed while simultaneously rotating the trocar around the axis thereof, in such a manner that the trocar can ablate the abdominal wall tissues. However, since this is a mechanical punctuation operation, the force the operator has to apply to the trocar for punctuation is very large, which is a burden to the operator. In addition, since the abdominal wall is often extended during the puncturing operation of the trocar, the puncturing operation may take time.

According to Patent Documents 1 and 2, moreover, a hard mirror has to be inserted through an inner needle for making preparations, and focusing adjustment has to be made. These operations inevitably take time. The hard mirror includes portions that are more projected rearward (i.e., toward the proximal end) than the inner needle. This being so, where the hard mirror is assembled in the trocar, the weight balance of the trocar becomes poor, and the trocar cannot be operated with ease.

When the trocar is operated to punctuate the abdominal wall, it is general that the initial punctuation into the body cavity (so-called the first trocar) is executed blindly. It is therefore important to prevent the trocar from touching organs in the body cavity or giving damage to them. On the other hand, where high-frequency current or ultrasonic waves are used to reduce the force the trocar applies to the abdominal wall for punctuation, as disclosed in Patent Documents 3 and 4, it is desirable to stop the supply of electric energy or ultrasonic energy when the trocar has just passed through the abdominal wall. However, the output control that enables stopping the supply of the electric energy or ultrasonic energy when the trocar has just passed through the abdominal wall, is not easy in practice. In many cases, therefore, the trocar is used when the body cavity is being observed by use of an endoscope. The high-frequency current and the ultrasonic waves are seldom used at the time of the first punctuation.

US 5,674,184 discloses a trocar obturator including a hollow tube having a substantially solid, optically clear tip used to insert a cannula through a tissue wall of a body cavity. The tip is formed of two opposing angularly oriented surface which converge to a line at the distal end of the tip. A cutting element is disposed along the line of intersection of the two angular surfaces, for example, an electrode. A viewing ' rod may be positioned within an axial bore from the proximal end of the trocar to the distal end of the tip. A viewing endoscope may be inserted into the viewing rod. The viewing rod may extend beyond the distal cutting end of the tip so as not to allow the cutting tip instrument to interfere with the image obtained by the viewing endoscope. Alternatively, an endoscope may be inserted so as to abut the clear distal tip, so that the surgeon may view the tissue through the clear tip.

EP 0624346 A2 describes a blunt-tipped ultrasonic trocar, having a feedback mechanism which senses a variation in load on the trocar tip and adjusts the ultrasonic energy level of the vibrating tip in accordance with the load. The blunt ti^{p} prevents injury, while the ultrasonic energy enables tissue penetration with less force. The feedback element may be an acoustical impedance feedback system, a piezoelectric element, a pressure detector or strain gauge, or a tissue hardness sensor.

EP 0761171 A2 describes an system for evulsing subcutaneous tissue having a transparent supersonic sheath and a viewing endoscope inserted therein.

The present invention has been made in consideration of the above circumstances, and an object of the present invention is to provide a trocar which enables reduction of a punctuation force, does not extend the abdominal wall or membranes, and enables observation of the state where the abdominal wall is punctuated.

Another object of the present invention is to provide a trocar which enables easy handling of observation means which is used at the time of punctuation.

Still another object of the present invention is to provide a trocar which is suitably used as the first trocar whose punctuation force is weak.

These problems are addressed by a trocar as defined in claim 1.

A trocar according to an aspect of the present invention comprises: an inner needle unit including optical observing means; and puncture-assisting means provided on an outer peripheral face of the inner needle unit, for assisting body wall puncture of the inner needle unit, wherein the puncture-assisting means has at least one of ultrasonic energy applying means and electric energy applying unit.

It is preferable that the ultrasonic energy applying means and the electric energy applying means each have a center axis line, and the center axis line is disposed concentrically with a center axis line of the inner needle unit.

It is preferable that the ultrasonic energy applying means has a tubular probe vibrating ultrasonically, and a supporting member holding a distance between the inner needle unit and the probe is provided at a node position of the ultrasonic vibration of the probe.

It is preferable that the supporting member is an elastic member, and the elastic member is fixed to an outer peripheral face of the inner needle unit so as not to be detached from the outer peripheral face against frictional resistance generated at inserting and withdrawing times of the inner needle unit.

The inner needle unit has a slipping protecting member with low friction coefficient near a distal end thereof.

It is preferable that the electric energy applying means has a tubular member made from metal material, and the tubular member has an insulating skin layer maintaining insulation against the inner needle unit on an inner peripheral face thereof and has an insulating skin layer on an outer peripheral face.

It is preferable that the tubular member is configured by arranging a plurality of tubular metal members having different inner diameters and outer diameters coaxially and providing an insulating layer between the respective tubular metal members.

It is preferable that the optical observing means is an image pickup device unit including an image pickup device and a circuit attached thereto, and the image pickup device unit is provided at an eccentric position from the center axis line of the inner needle unit near a distal end of the inner needle unit.

It is preferable that the inner needle unit is provided near a distal end thereof with illuminating means positioned in parallel with the image pickup device unit.

It is preferable that second optical observing means is provided outside the ultrasonic energy applying means.

It is preferable that the trocar comprises an image pickup section that picks up an image obtained by the optical observing means, a second image pickup section that picks up an image obtained by the second optical observing means, and image processing means that is connected to the image pickup section and the second image pickup section, respectively, and synthesizes an image from the image pickup section and an image from the second image pickup section to display the images on the same screen.

It is preferable that the second optical observing means is formed in an annular shape surrounding an outer periphery of the optical observing unit.

A trocar according to another aspect of the present invention comprises: an inner needle that is punctured and inserted into a body cavity wall; and a cannula in which the inner needle is removably inserted and which is inserted and placed in the body cavity wall, wherein the inner needle has a puncture needle that is transmitted with vibration energy or electric energy to puncture the body cavity wall and a sheath that allows insertion of the puncture needle on a center axis thereof, and the sheath has observation optical means and illuminating means emitting illumination light for illuminating a subject that are arranged around a distal end portion of the puncture needle.

It is preferable that the trocar comprises an operation section that operates a distal end portion of the puncture needle so as to protrude and retract the same to a distal end of the sheath.

A further trocar comprises: an inner needle that is punctured and inserted into a body cavity wall; and a cannula in which the inner needle is removably inserted and which is inserted and placed in the body cavity wall, wherein the inner needle has a puncture needle that is transmitted with vibration energy or electric energy to puncture the body cavity wall and a sheath that allows insertion of the puncture needle on a center axis thereof, and the puncture needle includes observation optical means and illuminating means that emits illumination light for illuminating a subject at a distal end portion thereof.

It is preferable that the observation optical means includes an image pickup device.

It is preferable that the puncture needle has a thin plate-shaped distal end portion.

It is preferable that the observation optical means is attached at a distal end portion of the puncture needle on a center axis of the puncture needle and the illuminating means is disposed around the observation optical means.

It is preferable that the sheath has a conical portion at a distal end face thereof, and the conical portion includes a slit portion serving as an insertion hole for the puncture needle and a plurality of blade portions projecting from a surface of the conical portion.

It is preferable that the distal end portion of the puncture needle is attached with the observation optical means and the illuminating means via an elastic member positioned at a node position of ultrasonic vibration.

It is preferable that the puncture needle has an insertion hole at an axial center portion thereof, and the observation optical means and the illuminating means have signal cables inserted and arranged in the insertion hole.

It is preferable that the sheath has an insertion hole on a wall portion thereof, and the observation optical means and the illuminating means have signal cables inserted and arranged in the insertion hole.

A further trocar comprises: an inner needle that is inserted into a body cavity wall in a puncture manner; and a cannula in which the inner needle can be removably inserted on a center axis of the cannula and which is inserted and placed in the body cavity wall, wherein the inner needle comprises a puncture needle that is transmitted with vibration energy or electric energy to puncture the body cavity wall and a sheath that allows insertion and withdrawal of the puncture needle on a center axis thereof, and the sheath includes a unit provided with observation optical means and illuminating means that emits illumination light and disposed inside a distal end portion of the sheath at a central position thereof.

It is preferable that the observation optical means includes an image pickup device disposed at a distal end of the puncture needle.

It is preferable that the sheath is attached with the observation optical means and the illuminating means via an elastic member.

It is preferable that the unit provided with the observation optical means and the illuminating means is attached inside the distal end of the sheath without including any gap between the unit and the sheath.

It is preferable that the sheath has a conical portion at a distal end face thereof, and the conical portion includes a slit portion serving as an mounting portion for the puncture needle and a plurality of blade portions projecting form a surface of the conical portion.

It is preferable that the trocar comprises an operation section that operates a distal end portion of the puncture needle so as to protrude and retract the same to a distal end of the sheath.

The invention can be more fully understood from the following detailed description when taken in conjunction with the accompanying drawings, in which:
FIG. 1 is a schematic configuration diagram of a whole trocar system;
FIG. 2 is a vertical sectional view of an inner needle unit of the trocar according to FIG. 1;
FIG. 3 is a side view showing an aspect where the trocar according to FIG. 1 has punctured an abdominal wall;
FIG. 4 is a front view of a monitor showing a state that the aspect where the trocar according to FIG. 1 has punctured the abdominal wall has been displayed on a monitor image;
FIG. 5 is a perspective view showing the vicinity of a distal end of an inner needle unit of a trocar according to the present invention;
FIG. 6 is a sectional view of a distal end taper portion of the inner needle unit of the trocar according to FIG. 5, taken along line VI-VI in FIG. 5;
FIG. 7 is a sectional view of the distal end taper portion, taken along line VII-VII in FIG. 6;
FIG. 8 is a sectional view of the distal end taper portion, taken along line VIII-VIII in FIG. 6;
FIG. 9 is a vertical sectional view of the vicinity of a distal end of a trocar showing a modification of the embodiment according to FIG. 5;
FIG. 10 is a side view showing a trocar;
FIG. 11 is a perspective view showing the vicinity of a distal end of an inner needle unit of the trocar according to FIG. 10;
FIG. 12 is a perspective view of a main section showing a suction port for suction provided on a cap near the distal end portion of the trocar according to FIG. 10;
FIG. 13 is a vertical sectional view of a main portion showing an annular objective optical system of an objective optical system of a rigid endoscope
FIG. 14A is a front view of a monitor showing a state that a monitor image has been displayed by a trocar according to FIG. 13;
FIG. 14B is a block diagram showing a transmission rute of an image signal of an observation image of the trocar according to FIG. 13;
FIG. 15A is a schematic configuration diagram of a main section for explaining a structure of a distal end portion of a trocar;
FIG. 15B is a cross sectional view showing a sectional structure of bipolar sheath of the trocar according to FIG. 15A;
FIG. 16A is a schematic configuration diagram of a main section for explaining a structure of a distal end portion of a trocar;
FIG. 16B is a cross sectional view showing a sectional structure of monopolar sheath of the trocar according to FIG. 16A;
FIG. 17 is a schematic configuration diagram of a main section for explaining a structure of a distal end portion of a trocar;
FIG. 18 is a vertical sectional view for explaining an internal structure of a trocar;
FIG. 19 is a front view of the trocar according to FIG. 18, viewed from a distal end of the trocar;
FIG. 20 is a sectional view of the trocar, taken along line 20-20 in FIG. 19;
FIG. 21 is a schematic configuration view of a whole trocar;
FIG. 22 is a vertical sectional view showing an internal structure of the trocar according to FIG. 21;
FIG. 23 is a front view showing a distal end face of the trocar according to FIG. 21;
FIG. 24 is a sectional view of the trocar, taken along line 24-24 in FIG. 23;
FIG. 25 is a sectional view of the trocar, taken along line 25-25 in FIG. 23;
FIG. 26 is a sectional view of the trocar, taken along line 26-26 in FIG. 25;
FIG. 27 is a side view showing an aspect where the trocar according to FIG. 21 has punctured an abdominal wall;
FIG. 28 is a front view showing a monitor screen on which an aspect where the trocar according to FIG. 21 has punctured an abdominal wall has been displayed;
FIG. 29 is a vertical sectional view of a main section showing a modification of the trocar according to FIG. 21;
FIG. 30 is a sectional view of the trocar, taken along line 30-30 in FIG. 29;
FIG. 31 is a front view showing a distal end face of a trocar;
FIG. 32 is a sectional view of the trocar, taken along line 32-32 in FIG. 31;
FIG. 33 is a sectional view of the trocar, taken along line 33-33 in FIG. 31;
FIG. 34 is a sectional view of the trocar, taken along line 34-34 in FIG. 33;
FIG. 35 is a front view showing a distal end face of a trocar;
FIG. 36 is a vertical sectional view of the trocar, taken along line 36-36 in FIG. 35;
FIG. 37 is a vertical sectional view of the trocar, taken along line 37-37 in FIG. 35; and
FIG. 38 is a vertical sectional view of the trocar, taken along line 38-38 in FIG. 37.

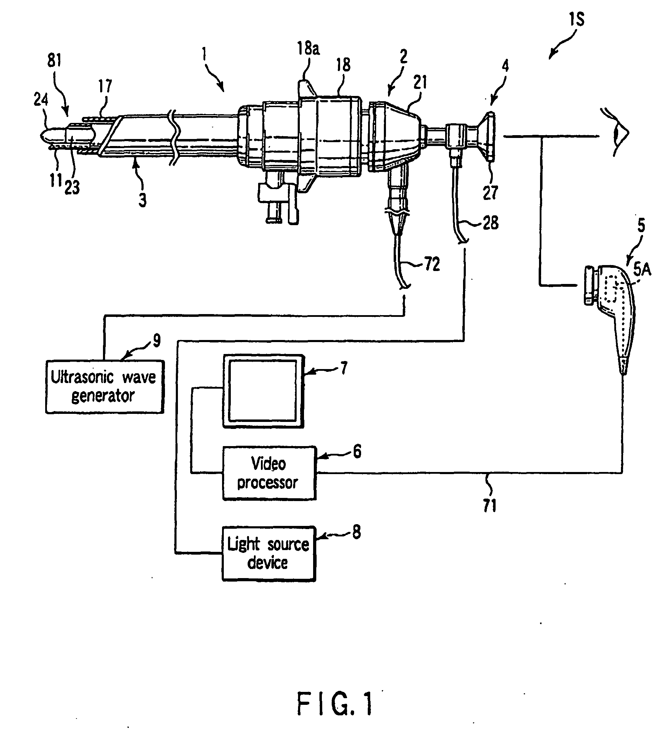

A first trocar will be explained below with reference to FIG. 1 to FIG. 4. FIG. 1 shows a schematic configuration of a whole trocar system 1S according to the embodiment. In FIG. 1, the trocar system 1S includes a trocar 1 and a peripheral device thereof. The trocar 1 has an inner needle unit 2 that punctures an abdominal wall and a cannula 3 placed in the abdominal wall. The inner needle unit 2 is removably inserted into the cannula 3. The inner needle unit 2 is attachably and detachably assembled with a rigid endoscope 4 described later.

The trocar system 1S further includes a camera head unit 5, a video processor 6, a monitor 7, a light source device 8, and an ultrasonic generator 9. The light source device 8 is connected to the rigid endoscope 4 via a light guide cable 28. The light source device 8 supplies illumination light to the rigid endoscope 4.

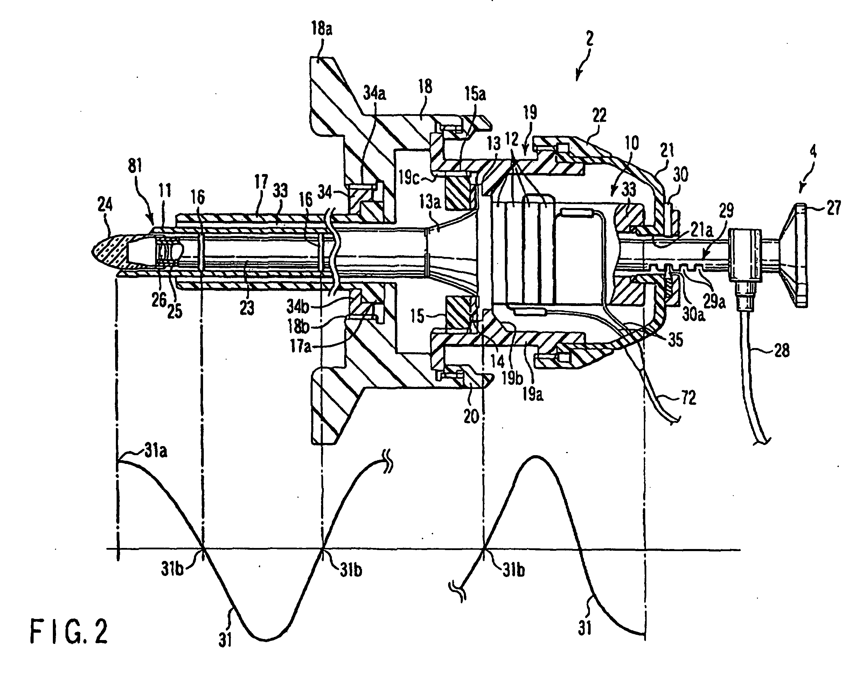

FIG. 2 is a sectional view of the inner needle unit 2. In FIG. 2, the cannula 3 is omitted. The inner needle unit 2 according to the embodiment mainly includes a bolded Langevin transducer 10, a probe 11, a sheath 17, a grip 18 grasped by an operator, a casing 19, and a cover 21.

The transducer 10 includes a plurality of piezoelectric elements 12 and a flange 13. A terminal-like electrode is sandwiched between the respective piezoelectric elements 12. One end portion of a lead wire 35 is soldered to the terminal-like electrode. The other end portion of the lead wire 35 passes through an electric cable 72 to be connected to the ultrasonic generator 9.

The ultrasonic generator 9 is connected to the inner needle unit 2 via the electric cable 72. The ultrasonic generator 9 generates a drive current for ultrasonic vibration at the transducer 10 at a puncture time of the trocar 1. The drive current from the ultrasonic generator 9 is supplied to the transducer 10 via the lead wire 35. The transducer 10 converts the drive current from the ultrasonic generator 9 to ultrasonic vibrations.

A horn 13a for amplifying the ultrasonic vibrations is provided at a distal end of the flange 13. The probe 11 made from titanium alloy is joined to a distal end of the horn 13a by a screw (not shown). Thereby, an ultrasonic procedure tool (ultrasonic energy applying means) 81 that transmits the ultrasonic vibrations of the transducer 10 that have been amplified by the horn 13a to the probe 11 and performing ultrasonic procedure on a body tissue by a distal end portion of the probe 11. Puncture-assistant means for assisting puncture of the inner needle unit 2 into a body wall is formed of the ultrasonic procedure tool 81.

The probe 11 and the transducer 10 are formed at central portions thereof with a conduit line 33 extending through the probe 11 and the transducer 10. That is, the probe 11 and the transducer 10 are formed in cylindrical bodies. The rigid endoscope 4 described later is removably inserted inside the conduit line 33.

The casing 19 includes a cylindrical casing main body 19a accommodating the transducer 10. A ring-like abutting portion 19b provided so as to project inwardly is provided on an inner peripheral face of the casing main body 19a.

A female screw portion 19c for oscillation fixation is formed on an inner peripheral face of the casing 19 ahead of the abutting portion 19b. A male-screw portion 15a on an outer peripheral face of a transducer-fixing ring 15 is attachably and detachably screwed to the screw hole 19c. The transducer 10 is disposed in a state that a rear face of the flange 13 is caused to abut on the abutting portion 19b. A ring-like rubber plate 14 is disposed on the side of a front face of the flange 13. In this state, the male-screw portion 15a of the transducer-fixing ring 15 is screwed into the female screw portion 19b of the casing 19. Thereby, the transducer 10 is fixed to the casing 19 via the rubber plate 13 by the transducer-fixing ring 15 in a state that the rear face of the flange 13 has been caused to abut on the abutting portion 19b of the casing 19.

Incidentally, a characteristic curve 31 in FIG. 2 shows a distribution of ultrasonic vibration. In the characteristic curve 31, reference numeral 31a denotes an antinode position of vibration and 31b denotes a node position of vibration, respectively. As shown in FIG. 2, the flange 13 is disposed at the node position on the distribution of ultrasonic vibration.

The sheath 17 is disposed so as to cover an outer peripheral face of the probe 11 over its entire length. A flange portion with a large diameter 17a is formed on an end portion of the sheath 17 on a near side thereof. A portion of the flange portion 17a of the sheath 17 is coupled to the grip 18.

A finger fitting portion 18a is formed on an outer peripheral face of a front end portion of the grip 18. A sheath-fixing screw hole 18b is formed at a central portion of a front end face of the grip 18. A sheath fixing ring 34 is attachably and detachably screwed into the screw hole 18b. A male screw portion 34a screwed to the screw hole 18b is formed on an outer peripheral face of the sheath-fixing ring 34. An engagement recess 34b engaged with a portion of the flange portion 17a of the sheath 17 is formed on an inner peripheral face of the sheath-fixing ring 34. The male screw portion 34a of the sheath-fixing ring 34 is screwed into the screw hole 18b of the grip 18 in a state that the engagement recess 34b of the sheath-fixing ring 34 has been engaged with the portion of the flange portion 17a of the sheath, so that the portion of the flange portion 17a of the sheath 17 is screwed and fixed to grip 18 by the fixing ring 34.

A front end portion of the casing 19 is screwed and fixed to the grip 18 via a casing-fixing ring 20. A front end portion of the cover 21 covering a proximal end of the transducer 10 is screwed and fixed to a cover-fixing ring 22 on a near side of the casing 19.

An opening portion 21a for inserting the rigid endoscope 4 is formed near the center axis of the cover 21. A lock button 30 serving as a mechanism for fixing the rigid endoscope 4 in a peripheral edge portion of the opening portion 21a at a proper position in an axial direction of the rigid endoscope 4 and releasing the rigid endoscope 4 is provided at a rear end portion of the cover 21. The lock.button 30 includes a fitting protrusion 30a.

The rigid endoscope 4 includes a shaft-like slender endoscope insertion portion 23. A plurality of rubber linings (elastic members) 16 are provided on an outer peripheral face of the endoscope insertion portion 23. Regarding the characteristic curve 31 of the distribution of ultrasonic vibration of the probe 11, the rubber linings 16 are formed integrally with the endoscope insertion portion 23, especially, at respective portions corresponding to the node positions 31b of vibration. Thereby, the rubber linings 16 are fixed on the outer peripheral face of the endoscope insertion portion 23 so as not to be detached against frictional resistance of insertion and withdrawal of the endoscope insertion portion 23. The inner peripheral face of the probe 11 of the transducer 10 and the outer peripheral face of the endoscope insertion potion 23 of the rigid endoscope 4 are arranged on the same axial line so as to be spaced from each other by a fixed distance by the rubber linings 16 of the endoscope insertion portion 23.

A lock groove 29 is provided on an outer peripheral face of a near side end portion of the endoscope insertion portion 23 of the rigid endoscope 4. The lock groove 29 includes a plurality of engagement recesses 29a provided in parallel along an insertion axis direction of the endoscope insertion portion 23. The fitting protrusion 30a of the lock button 30 is biased so as to engage either one of the engagement recesses 29a of the lock groove 29 by an elastic member. The rigid endoscope 4 is fixed at any position in the axial direction in a state that the fitting protrusion 30a of the lock button 30 and either one of the engagement recesses 29a of the lock groove 29 have been engaged with each other. According to a pushing-in operation of the lock button 30 against a biasing force of the elastic member, the fitting protrusion 30a of the lock button 30 is moved in a direction in which the fitting protrusion 30a of the lock button 30 is disengaged from the engagement recess 29a so that position fixing of the rigid endoscope 4 in the axial direction is released.

A distal end portion of the endoscope insertion portion 23 is provided with an objective optical system 26 serving as the optical observing means and a light guide 25. A relay lens (not shown) transmitting an image captured by the objective optical system 26 and a light guide 25 that is a light guide fiber (not shown) transmitting illumination light are disposed inside the endoscope insertion portion 23.

One end portion of a light guide cable 28 is connected to a near side of the rigid endoscope 4. The other end portion of the light guide cable 28 is connected to the light source device 8. Thereby, illumination light from the light source device 8 is transmitted to the rigid endoscope 4.

A puncture cap 24 is attached at a distal end of the rigid endoscope 4. The cap 24 is formed from transparent glass material or transparent resin. Tissue is peeled off by the cap 24 at a time of abdominal wall puncture performed by the trocar 1.

An eyepiece optical system (not shown) including an eyepiece 27 is configured on the near side of the rigid endoscope 4. A view field of the endoscope 4 is illuminated by illumination light emitted from the light guide 25 at a puncture time of the trocar 1. The rigid endoscope 4 takes an aspect of puncture into an abdominal wall within an observation field via the cap 24. Thereby, the view field at the puncture time of the trocar 1 is taken from the objective optical system 26. An observation image obtained by the objective optical system 26 is focused onto the eyepiece 27 and it is further transmitted from the eyepiece 27 to an operator.

The camera head unit 5 is attachably and detachably attached to the rigid endoscope 4. The camera head unit 5 is connected to the video processor 6 via a cord 71. The monitor 7 is connected to the video processor 6. An image pickup device 5A converting an observation image obtained by the endoscope 4 to an image signal is incorporated in the camera head unit 5. The video processor 6 converts the image signal from the image pickup device 5A of the camera head unit 5 to a video signal. The monitor 7 displays the video signal from the video processor 6 as an image.

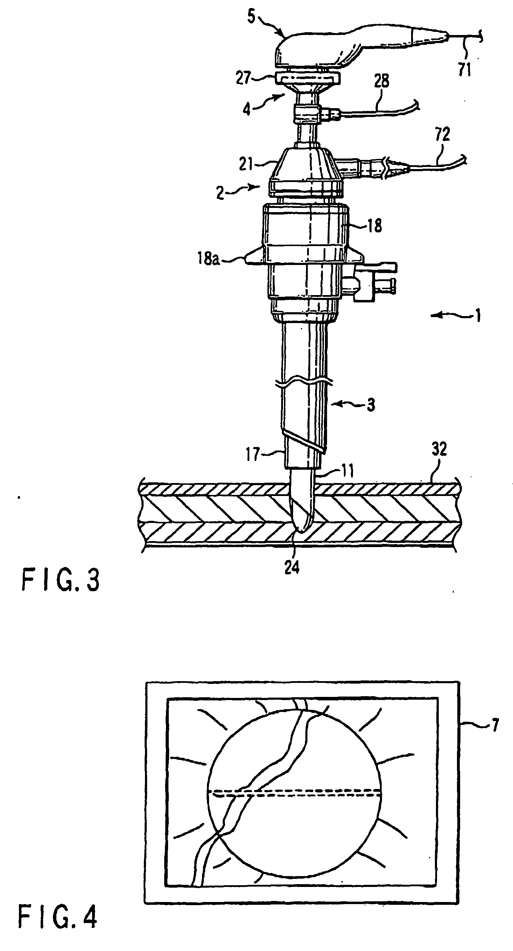

Next, an operation of the configuration will be explained. FIG. 3 shows an aspect that the trocar 1 according to the embodiment is puncturing an abdominal wall 32 and FIG. 4 shows an image during a puncture operation displayed on the monitor 7.

Before the trocar system 1S is used, the inner needle unit 2 is first inserted into the tube of the cannula 3. The inner needle unit 2 and the cannula 3 are assembled integrally by a lock mechanism (not shown) so that the trocar 1 is fixed in a state that they have formed the trocar 1.

Subsequently, the rigid endoscope 4 is inserted into the conduit line 33 in the inner needle unit 2. At this time, the lock button 30 is operated at a position where the cap 24 at the distal end of the rigid endoscope 4 projects from the probe 11. Thereby, the fitting protrusion 30a is fitted into the engagement recess 29a at a proper position of the lock groove 29, so that the position of the inner needle unit 2 and the rigid endoscope 4 in the axial direction is fixed. Incidentally, the position of the inner needle unit 2 and the rigid endoscope 4 in the axial direction at a time of abdominal wall puncture (the fitting position between the fitting protrusion 30a and the engagement recess 29 of the lock groove 29) always becomes the same position for securing an optimal view field frequently.

Thereafter, the cable 72 supplying drive current to the inner needle unit 2 is connected to the ultrasonic generator 9. The light guide cable 28 supplying illumination light to the rigid endoscope 4 is connected to the light source device 8. An image obtained from the rigid endoscope 4 may be directly observed through the eyepiece 27 by the naked eye. Generally, the camera head unit 5 is connected to the eyepiece 27 so that an image obtained from the rigid endoscope 4 is displayed on the screen of the monitor 7. The trocar system 1S is frequently operated while the screen of the monitor 7 is being observed.

After the above-described preparation, an operator places his/her finger on the finger fitting portion 18a of the grip 18 to grasp the inner needle unit 2. In this state, the operator brings the cap 24 of the inner needle unit 2 in contact with a site where a skin has been preliminarily opened (a skin-opened site) and pushes the cap to such an extent that the cap 24 is buried in the site. Thereafter, the operator pushes the distal end portion of the probe 11 in such an extent that the distal end portion is brought in contact with a subcutaneous tissue. At this time, illumination light from the light source device 8 is illuminated on a contact face through the light guide cable 28 and the light guide 25 by the cap 24. Thereby, an image of a skin-opened portion contacting with the cap 24 is observed through the objective optical system 26 by the operator. The observed image is converted to a signal through the eyepiece 27 by the image pickup device of the camera head unit 5. An image signal outputted from the image pickup device is fed to the video processor 6 to be converted to a video signal and be displayed on the monitor 7.

In this state, the operator pushes the inner needle unit 2 into the abdominal wall while repeating a twisting motion for forwardly and reversely rotating the inner needle unit 2 about its center axis. Thereby, the operator conducts a puncture operation for puncturing the trocar 1 into the abdominal wall 32. The operator performs a stepping-in operation of a footswitch (not shown) during the operation to drive the ultrasonic generator 9. At this time, drive current from the ultrasonic generator 9 is supplied from the terminal-like electrode via the lead wire 35 to each element 12 of the transducer 10. Thereby, the drive current is converted to ultrasonic vibration by the transducer 10 so that ultrasonic wave outputting is performed. The ultrasonic vibration is amplified by the horn 13a to reach the probe 11.

In addition to the repetitive twisting operation of the trocar 1 and the pushing-in operation thereof conducted by the operator when he/she conducts the puncture operation of the trocar 1 into the abdominal wall 32, especially, the ultrasonic vibration of the probe 11 significantly contributes to expansion of a diameter of a punctured hole. That is, a magnitude of the puncturing force required for the trocar 1 is considerably reduced, so that the operator can advance puncture of the trocar 1 remarkably smoothly.

The distal end of the probe 11 corresponds to the antinode position 31a on the ultrasonic vibration distribution. An image during puncture is displayed on the monitor 7, as shown in FIG. 4. Incidentally, since the probe 11 is generally vibrated ultrasonically, frictional heat is generated due to careless touch with the probe 11. In the embodiment, the probe 11 and the endoscope insertion portion 23 are supported so as to be spaced from each other by the rubber linings 16 at the node positions 31b of ultrasonic vibration. Therefore, the inner needle unit 2 can be operated without causing useless heat generation or abnormal noise between contact portions of the probe 11 and the endoscope insertion portion 23.

The operator can confirm an aspect where an operation of the trocar 1 puncturing the abdominal wall 32 advances via an image displayed on the monitor 7 during a series of puncture operations. That is, the operator can advance puncture conducted by the trocar 1 while confirming saphenous blood vessels in an abdominal wall, a muscular layer, or a peritoneal membrane on the screen of the monitor 7, and while also confirming presence/absence of organ adhesion in an abdominal cavity on the screen of the monitor 7. Of course, the ultrasonic wave output is stopped by stopping the operation of the footswitch (not shown). Therefore, a temporary suspension state can be achieved during puncturing conducted by the trocar 1.

In the embodiment, ribs and grooves fitted with the ribs for rotation prevention (not shown) are provided on abutting faces of the sheath 17 and the grip 18, abutting faces of the grip 18 and the casing 19, and abutting faces of the flange 13 and the casing 19. Therefore, respective portions of the inner needle unit 2 are loosened or dissolved unexpectedly when the repetitive twisting motion of the inner needle unit 2 is caused during puncturing of the trocar 1.

When the puncture operation of the trocar 1 is completed, the inner needle unit 2 together with the rigid endoscope 4 is pulled out of the cannula 3. Thereby, only the cannula 3 can be placed in its puncturing state into the abdominal wall 32, and the cannula 3 can be used as an insertion port through which an instrument is inserted into the abdominal cavity.

With the above configuration, the following effects can be achieved. That is, according to the configuration of the trocar 1 according to the embodiment, an operator can confirm an aspect that the cap 24 at the distal end of the inner needle unit 2 passes through the abdominal wall 32 during puncturing operation of the trocar 1 into the abdominal wall. Therefore, such a trouble as contact with a blood vessel or contact with an organ occurring at a puncture time of the trocar 1 into the abdominal wall 32 can be avoided so that high safety can be achieved.

Reduction of the magnitude of the puncture force required when the trocar 1 punctures the abdominal wall 32 can be achieved owing to ultrasonic vibration at a driving time of the transducer 10. Thereby, penetration into the abdominal wall can be conducted easily. Therefore, an operation for the trocar 1 to puncture the abdominal wall 32 can be performed considerably smoothly with a remarkably reduced magnitude of the puncture force. That is, the trocar 1 can be used as a first trocar driven with a small magnitude of puncture force.

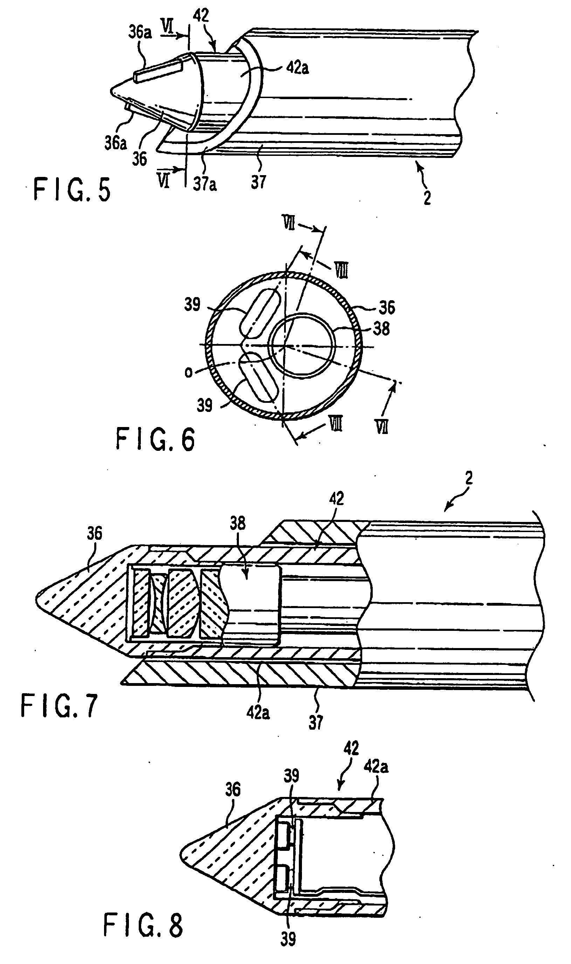

FIG. 5 to FIG. 8 show an embodiment of the present invention. The embodiment is a modification of the trocar 1 according to FIG. 1 to FIG. 4, and only parts or sections of the embodiment modified from the embodiment of FIGS. 1 to 4 will be explained.

FIG. 5 shows the vicinity of a distal end of an inner needle unit 2. A distal end acute angle portion 37a sharpened at an acute angle shape by cutting both left and right sides of the distal end portion of the probe 11 according to FIGS. 1 to 4 is formed at a distal end portion of a probe 37 of the inner needle unit 2 according to FIGS. 1 to 8.

An insertion portion 42a of a viewing inner needle 42 having an incorporated image pickup device is inserted in the probe 37. FIG. 6 is a sectional view of the vicinity of a distal end tapered portion of the insertion portion 42a of the viewing inner needle 42, taken along line VI-VI in FIG. 5. One image pickup device unit 38 (shown in FIG. 7) and two LEDs (light emitting diodes) 39 (shown in FIG. 8) are disposed at a distal end of viewing inner needle 42. The image pickup device unit 38 is incorporated with an image pickup device such as a CMOS type (complementary metal-oxide semiconductor) image sensor or a CCD type (charge-coupled device or the like) image sensor, and a circuit board and a lens group attached to the sensor. Two LEDs 39 are used instead of the light guide 25 in the embodiment of FIGS. 1 to 4.

A conical cap 36 made from transparent material is disposed at a distal end of viewing inner needle 42 so as to be firmly coupled thereto as an exclusive member. FIG. 7 is a sectional view of the vicinity of joined faces of the cap 36 and the image pickup device unit 38, taken along line VII-VII in FIG.6 especially showing the vicinity.

Two blade-like protrusions 36a are provided on a conical outer peripheral face of the cap 36. FIG. 8 is a sectional view of the vicinity of the joined faces, taken along line VIII-VIII in FIG. 6. As also apparent from FIG. 6, the center line of the image pickup device unit 38 is disposed at a position slightly deviated from the center axis 0 of the insertion portion 42a of viewing inner needle 42. Thereby, an arrangement space for two LEDs 39 can be secured inside the insertion portion 42a of viewing inner needle 42.

Next, an operation of the trocar 1 according to the embodiment including viewing inner needle 42 thus configured will be explained. An abdominal wall puncture portion contacting with the cap 36 during an operation for the trocar 1 according to the embodiment to puncture an abdominal wall is illuminated by illumination light from two LEDs 39. An observation image of viewing inner needle 42 is further observed through the cap 36. The image of the inner needle for an incorporated image pickup device 42 is converted into a video signal by the image pickup device in the image pickup device unit 38 and the video signal is converted to a video image by the video processor 6 to be displayed on the monitor 7. Thereby, an operator can perform puncturing while observing the aspect that the puncture operation of the trocar 1 into the abdominal wall 32 advances on the screen of the monitor 7.

In the embodiment, the two blade-like protrusions 36a are provided on the conical outer peripheral face of the cap 36. Thereby, the operator can repeatedly perform twisting operation of the trocar 1 in forward and reverse rotational directions to peel off an abdominal wall tissue effectively.

According to the embodiment, the following effects can be achieved. In addition to the effect of the first embodiment, the cap 36 at the distal end and the objective optical system incorporated in the image pickup device unit 38 constitute an optical combination by using the dedicated viewing inner needle 42 having an incorporated image pickup device for an abdominal wall puncture. Thereby, focusing of viewing inner needle 42 is made unnecessary and excellent view field can be obtained.

Since the LED system is adopted for illumination in the embodiment, the light source device and the light guide cable are made unnecessary. Therefore, a whole configuration of the trocar system 1S is simplified and preparation is facilitated. Since an operation portion structure of viewing inner needle 42 is simple and small-sized, the trocar 1 is grasped easily and operation of the trocar 1 is made easy.

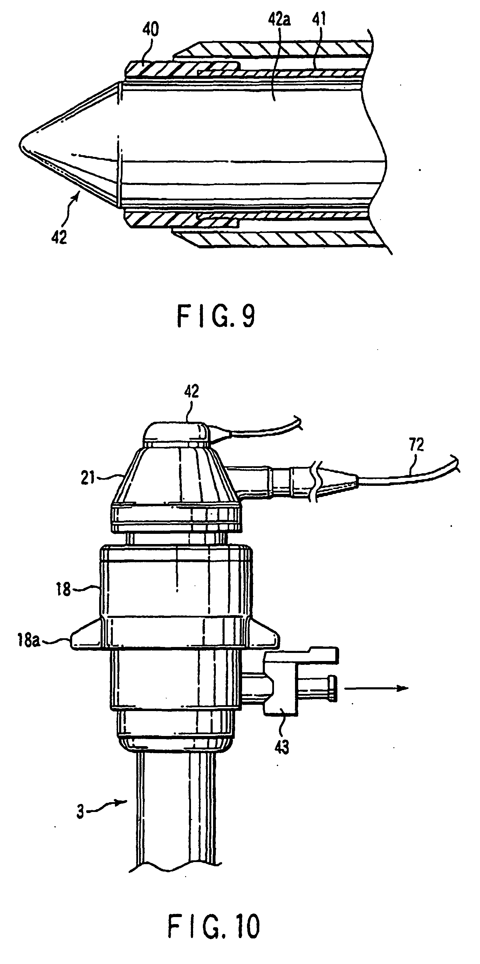

FIG. 9 shows a modification of the trocar 1 according to the embodiment of FIGS. 5 to 8. In the modification, a protective sheath 41 for protecting an insertion portion 42a of a viewing inner needle 42 having an incorporated image pickup device is provided. A protective member (a slipping protective member) 40 made from material with low friction coefficient such as polytetrafluoroethylene is provided at the distal end of the protective sheath 41. Thereby, friction heat generation and abnormal noise generation due to ultrasonic vibration to viewing inner needle 42 can be prevented. Therefore, early failure of viewing inner needle 42 can be prevented.

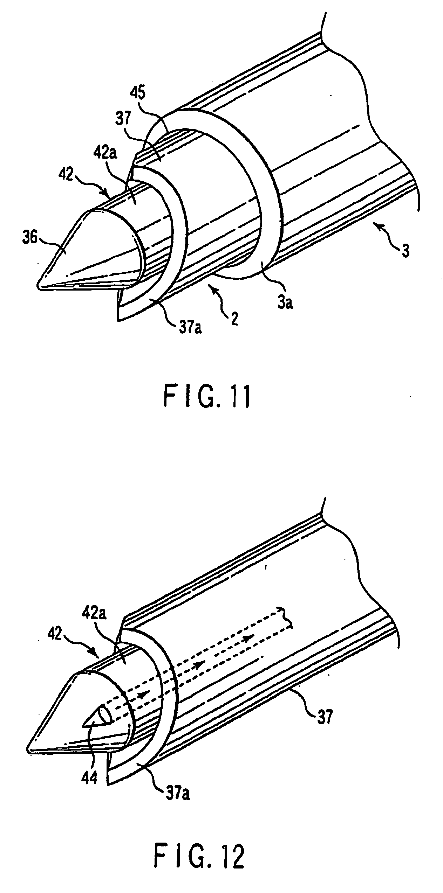

FIG. 10 and FIG. 11 show a further embodiment of the present invention. The embodiment is a modification of the trocar 1 according to FIG. 5 to FIG. 8, and only parts or sections of the embodiment modified from the embodiment of FIGS. 5 to 8 will be explained.

A suction cock 43 is provided at a proximal end portion of a cannula 3 in a trocar 1 according to the embodiment. FIG. 10 shows an aspect that suction pressure is being applied in a state that a suction tube (not shown) is connected to the suction cock 43 of the cannula 3. As a result, suction pressure can be applied near the distal end of an inner needle unit 2 through a clearance 45 between a probe 37 and a sleeve 3a of the cannula 3, as shown in FIG. 11.

When ultrasonic wave is outputted during a puncture operation of the trocar 1 into an abdominal wall 32, mist is generated from an abdominal wall tissue. In the configuration of the embodiment, the mist can be sucked from the clearance 45 between the probe 37 and the sleeve 3a of the cannula 3. Thereby, an excellent view field of a viewing inner needle 42 having an incorporated image pickup device can be assured.

Like a modification of the trocar 1 according to the embodiment shown in FIG. 12, a suction port 44 for suction may be provided on the cap 36 of viewing inner needle 42. A suction conduit line communicating with the suction port 44 is provided in an insertion portion 42a of viewing inner needle 42 so as to extend from the insertion portion 42a to an operation portion. A suction cock (not shown) is provided on the operation portion of viewing inner needle 42. An effect similar to that in the trocar 1 according to the third embodiment can be obtained by connecting a suction tube (not shown) to the suction cock.

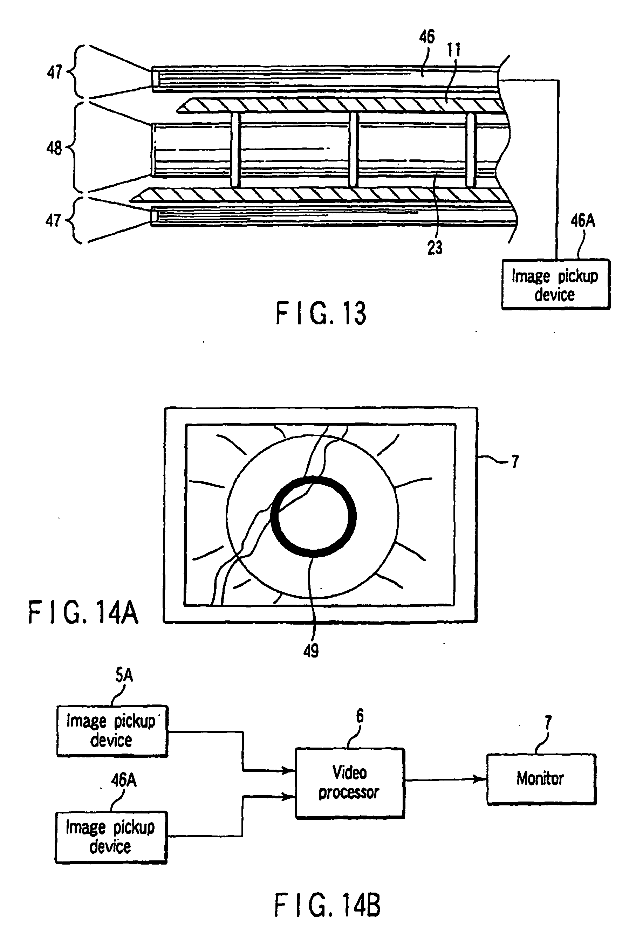

FIG. 13 and FIGS. 14A and 14B show a further trocar. The trocar is a modification of the trocar 1 according to FIG. 1 to FIG. 4, and only parts or sections of a trocar 1 modified from the trocar 1 according to FIGS. 1 to 4 will be explained here.

In the embodiment, an annular objective optical system (a second optical observing means) 46 is added to the inner needle unit 2 of the trocar 1 according to the embodiment, as shown in FIG. 13. The annular objective optical system 46 is disposed on an outer peripheral face of a probe 11. The annular objective optical system 46 is connected to an image pickup device 46A for converting an observation image to an image signal. As shown in FIG. 14B, the image pickup device 46A is connected to a video processor 6. Thereby, an annular view range 47 that is observed from the outside of the probe 11 can be obtained by the annular objective optical system 46.

With the above configuration, the following effect can be obtained. According to the configuration, in addition to the view range 48 similar to that of the objective optical system 26 of the rigid endoscope 4 of the trocar 1 of FIGS. 1 to 4, the annular view range 47 that is observed from the outside of the probe 11 can be obtained by the annular objective optical system 46. The annular view range 47 obtained by the image pickup device 46A of the annular objective optical system 46 and the view range 48 outputted from the image pickup device 5A of the camera head unit 5 according to the embodiment shown in FIGS. 1 to 4 and obtained by the rigid endoscope 4 are processed so as to form a composite image by an image processing device (not shown) in the video processor 6. Thereby, a portion except a shadow 49 of the probe 11 such as shown in FIG. 14A can be view-observed by the monitor 7. As a result, since a puncture site can be observed from the inside and the outside of the probe 11 vibrating ultrasonically, abdominal wall puncture of the trocar 1 can be performed more safely than the first embodiment. Therefore, the trocar according to the embodiment can be utilized as a first trocar further easily.

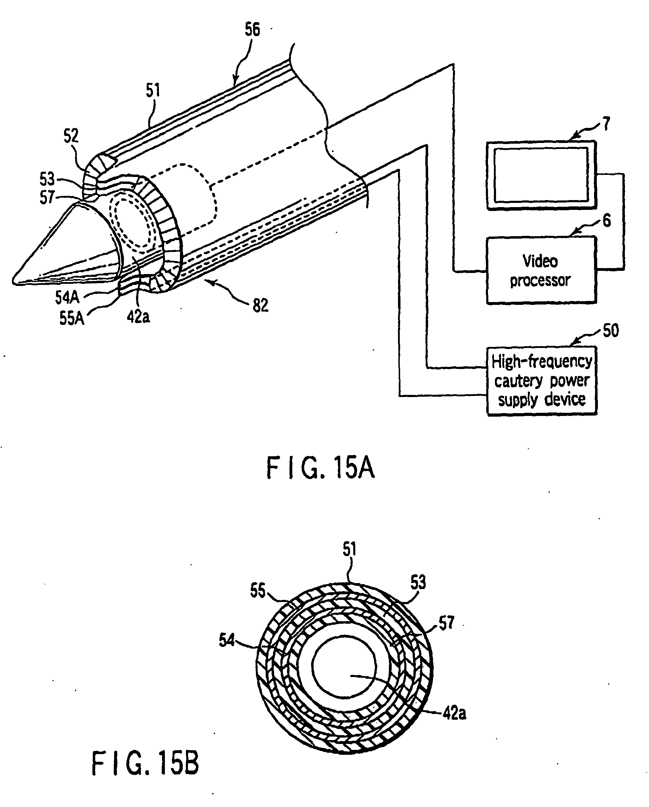

FIGS. 15A and 15B show a further embodiment of the present invention. The embodiment is a modification of the embodiment of FIG. 1 to FIG. 4 and the embodiment of FIG. 5 to FIG. 8. Here, only modified parts or sections of the embodiment different in configuration from the embodiments of FIGS. 1 to 8 will be explained.

In the embodiment, a viewing inner needle 42 having an incorporated image pickup device is used instead of the inner needle units 2 shown in the embodiments of FIGS. 1 to 8. Electric energy applying means is provided as puncture-assistant means for viewing inner needle 42 instead of the ultrasonic energy applying means including the probe 11 of an ultrasonic vibration system shown in the embodiments of FIGS. 1 to 8. As shown in FIG. 15A, in the embodiment, a bipolar procedure instrument 82 utilizing high-frequency current of a bipolar system is used as the electric energy applying means is used as the electric energy applying means.

The bipolar procedure instrument 82 includes a bipolar sheath 56 covering an outer peripheral face of an insertion portion 42a of a viewing inner needle 42 having an incorporated image pickup device. The bipolar sheath 56 includes an inner electrode 54A formed at a distal end of an inner pipe made from metal, in which high-frequency current flows, and an outer electrode 55A formed at a distal end of an outer pipe 55 made from metal, in which high-frequency current flows. As shown in FIG. 15B, an insulating layer 53 is disposed between the inner electrode 54A and the outer electrode 55A. An outer periphery of the outer electrode 55A is covered with an outer peripheral face insulating skin 51. An insulating layer 57 for securing insulation from the insertion portion 42a is provided inside the inner electrode 54A.

An insulating end face 52 is provided at a distal end of the bipolar sheath 56. Each electrode and an insulating layer come in close contact with each other. Thereby, such a configuration is obtained that opening/clotting high-frequency wave of a bipolar system is generated using two electrodes 54A and 55A utilizing high frequency current of bipolar system instead of the ultrasonic vibration system. The inner electrode 54A and outer electrode 55A are connected to a high-frequency cautery power source device 50 via a near side of the sheath 56. The high-frequency cautery power source device 50 generates opening/clotting high frequency wave of bipolar system.

During use of the trocar 1 with this configuration, opening high-frequency current of bipolar system is supplied from the high-frequency cautery power source device 50 to the inner electrode 54A and the outer electrode 55A in a state of view field observation from the distal end of viewing inner needle 42. At this time, an abdominal wall puncture of the trocar 1 based upon opening action can be performed by causing opening high-frequency current to act on an abdominal wall tissue between the inner electrode 54A and the outer electrode 55A.

Therefore, the following effect can be achieved in the embodiment. In the embodiment, abdominal wall puncture of the trocar 1 can be performed with a simple structure and a small magnitude of puncturing force as compared with the trocar 1 of the system utilizing ultrasonic vibration. Therefore, cost reduction of the whole trocar system 1S can be achieved. Even if bleeding occurs from an abdominal wall, hemostasis based upon bipolar clotting output can be performed within the view field so that the trocar 1 according to the embodiment is safe and can be used as a first trocar.

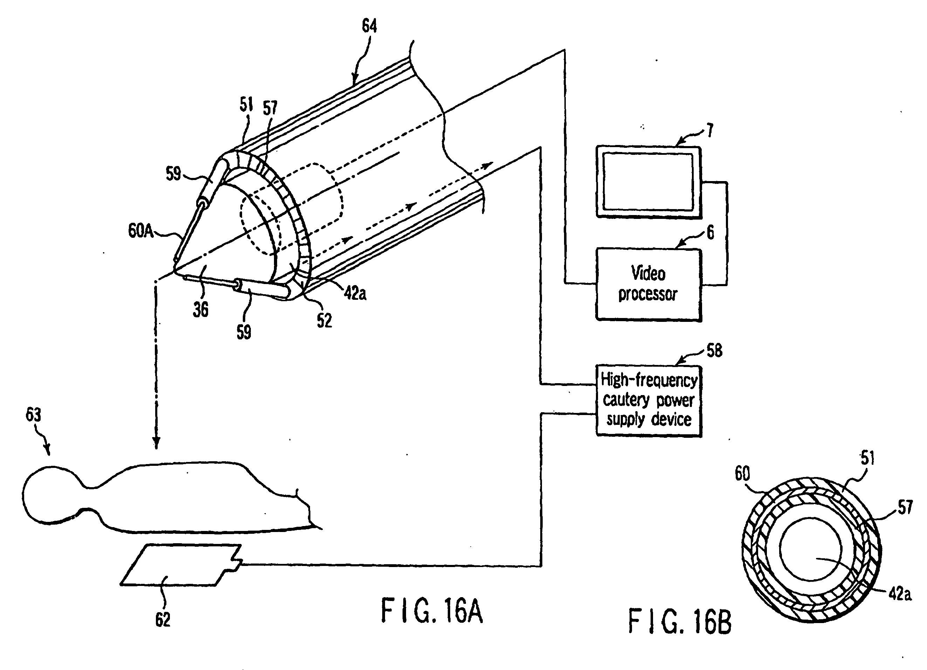

FIGS. 16A and 16B show a further embodiment of the present invention. The embodiment is a modification of the embodiment of FIGS. 15A and 15B, and only parts or sections of a trocar according to the embodiment modified from the trocar 1 according to FIGS. 15A and 15B will be explained.

That is, the configuration that two electrodes 54A and 55A utilizing high-frequency current of bipolar system are used is shown in the embodiment of FIGS. 15A and 15B, but an electrode utilizing high-frequency current of monopolar system instead of the bipolar system is used in this embodiment.

As shown in FIG. 16A, a monopolar sheath 64 covering an outer peripheral face of an insertion portion 42a of a viewing inner needle 42 having an incorporated image pickup device is provided in this embodiment. The monopolar sheath 64 includes an electrically conductive pipe, for example, an electrode 60A electrically connected to a pipe 60 made from metal. A distal end portion of the electrode 60A is formed in approximately V shape to be disposed near a distal end of a cap 36. Proximal end portions of the electrode 60A are covered with insulating coatings 59, thereby preventing high-frequency discharging to an unnecessary site. As shown in FIG. 16B, an outer peripheral face insulating skin 51 is formed on an outer peripheral face of the pipe made from metal. An inner insulating layer 57 securing insulation from an insertion portion 42a is formed on an inner peripheral face of the pipe made from metal. The pipe made from metal is sandwiched between the outer peripheral face insulating skin 51 and the inner insulating layer 57. An end face of the sheath 64 is kept insulating by an insulating end face 52, so that the electrode 60A and each insulating layer come in close contact with each other.

The metal-made pipe constituting the monopolar sheath 64 is connected to a high-frequency cautery power source device 58 for generating opening and clotting high frequency wave of monopolar via a near side of the metal-made pipe. A mating electrode plate 62 extending from the high-frequency cautery power source device 58 is caused to adhere on and contact with a patient 63.

During use of the trocar 1 thus configured, high-frequency opening and clotting current of monopolar is supplied from the high-frequency cautery power source device 58 to the electrode 60A in an observation state with a view field obtained through the cap 36 at the distal end of viewing inner needle 42. At this time, when operation current acts on an abdominal wall tissue from the electrode 60A, it is recovered in the high-frequency cautery power source device 58 from the patient 63 via the mating electrode plate 62. Thereby, abdominal wall puncture can be performed according a high-frequency opening action of the trocar 1. Excellent view field can be maintained by exhausting smoke generated during high-frequency output from a gap between the insertion portion 42a and the monopolar sheath 64 using a suction device (not shown).

Therefore, the following effect can be obtained in the embodiment. In the embodiment, abdominal wall puncture of the trocar 1 can be performed with a simple structure and a small magnitude of puncturing force as compared with the trocar 1 of the system utilizing ultrasonic vibration and the trocar 1 utilizing the electrode of bipolar system. Therefore, cost reduction of the whole trocar system 1S can be achieved. Even if bleeding occurs from an abdominal wall, hemostasis based upon bipolar clotting output can be performed within the view field so that the trocar 1 according to the embodiment is safe and can be used as a first trocar.

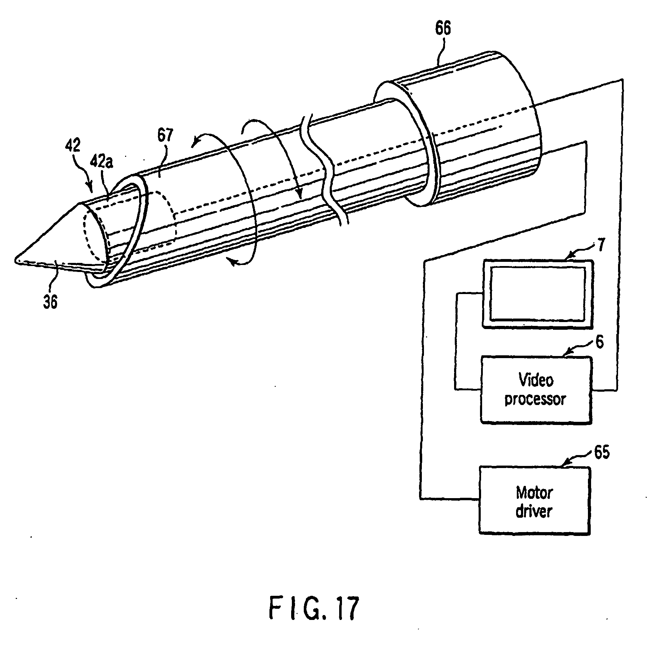

FIG. 17 shows a further embodiment of the present invention. This embodiment is a modification of the trocars 1 according to the embodiment of FIG. 1 to FIG. 4 and the embodiment of FIG. 5 to FIG. 8. Here, only modified parts or sections of the embodiment different in configuration from the embodiments of FIGS. 1 to 8 will be explained.

In the embodiment, a cylindrical puncture blade 67 is provided so as to cover an outer peripheral portion of an insertion portion 42a of a viewing inner needle 42 having an incorporated image pickup device instead of the ultrasonic vibration system according to the embodiments of FIGS. 1 to 4. A proximal end portion of the puncture blade 67 is coupled with a motor 66 that rotationally drives the puncture blade 67 about an axis of a center line thereof. The motor 66 is connected to a motor driver 65. Rotation control of the motor 66 is conducted by the motor driver 65.

During use of the trocar 1 thus configured, the puncture blade 67 is rotationally driven by the motor 66 in a state of observation with a view field obtained through the cap 36 at the distal end of viewing inner needle 42. At this time, abdominal wall puncture of the trocar 1 can be easily conducted by forward rotating motion and reverse rotating motion of the puncture blade 67 about the axis of the center line.

Such a configuration can be adopted that a mating electrode plate (not shown) is caused to adhere on a patient and high-frequency current is applied to the puncture blade 67 from a high-frequency cautery power source device. In this case, the puncture blade 67 can be used as a monopolar high-frequency opening procedure instrument. Therefore, puncture with reduced bleeding can be conducted, which results in safety. Incidentally, rotation of the motor 66 may be conducted in one direction, and forward and reverse rotations thereof may be repeated.

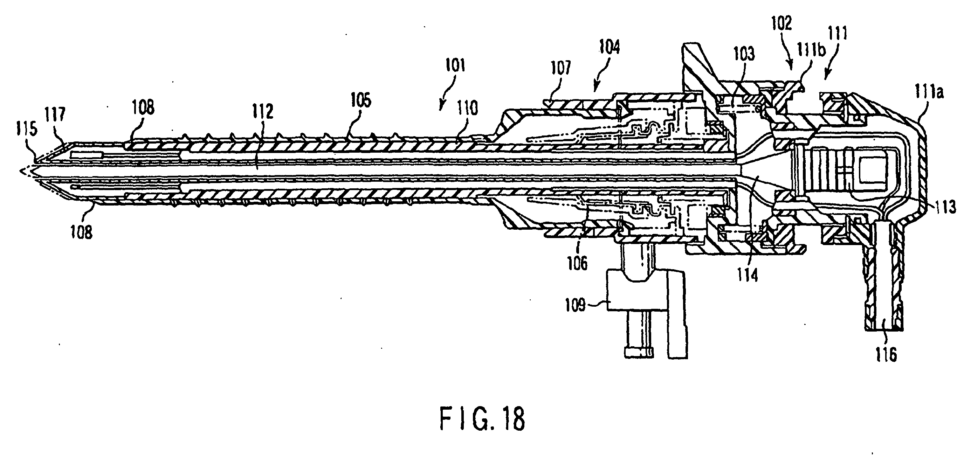

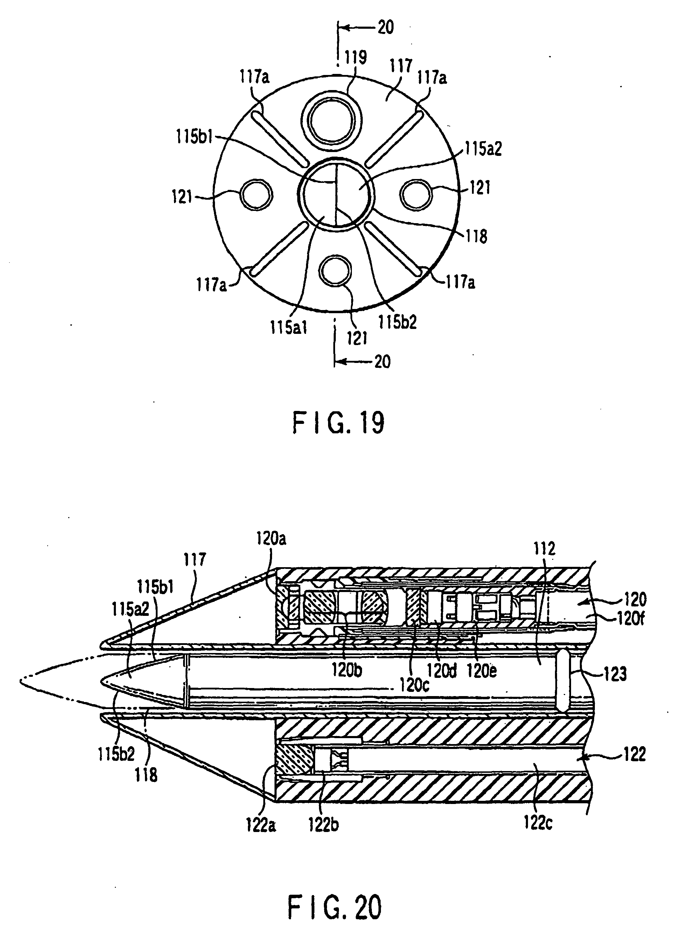

FIG. 18 and FIG. 20 show an embodiment of a trocar. FIG. 18 is an internal structure of a trocar 101 according to this embodiment. The trocar 101 is used in a state that two parts, namely, an inner needle unit 101 and a cannula 104, have been attachably and detachably assembled to each other. The cannula 104 includes a sleeve 105 and a head member 107. The sleeve 105 is placed and fixed to a body cavity wall. The head member 107 is incorporated with a valve 106 holding airtight state at an insertion or withdrawal of an endoscope or a surgery instrument (not shown).

A taper face 108 tapered is formed at a distal end portion of sleeve 105. An air-feeding mouth ring 109 for pneumoperitoneum gas communicating with an insertion hole of the sleeve 105 is provided in a projecting manner on the head member 107.

The inner needle unit 102 includes a sheath portion 110 and a grasping portion 111. The sheath portion 110 is a cover member that is inserted and arranged in the insertion hole of the cannula 104. The grasping portion 111 is coupled so as to be positioned at a proximal end portion of the sheath portion 110.

An approximately linear and slender rod-shaped ultrasonic probe 112 is inserted and arranged in the sheath portion 110 on a center axis of the sheath portion 110. The ultrasonic probe 112 transmits ultrasonic vibration. An ultrasonic transducer 113 and a horn 114 are provided inside the grasping portion 111. The ultrasonic transducer 113 generates ultrasonic vibration. The horn 114 is disposed at a distal end of the ultrasonic transducer 113. A distal end portion of the ultrasonic probe 112 is coupled to a distal end portion of the horn 114. Ultrasonic vibration from the transducer 113 is amplified by the horn 114 to be transmitted to the ultrasonic probe 112. As shown in FIG. 20, a probe distal end section 115 is provided at a distal end of the ultrasonic probe 112. The probe distal end section 115 is formed in an approximately conical shape tapered for puncturing a body cavity wall. As shown in FIG. 19, the probe distal end section 115 is sandwiched between two curved faces 115al and 115a2 and it is provided with two ridge lines 115b1 and 115b2 at symmetrical positions about a longitudinal center axis of the probe 112. A blade portion for cutting a body tissue is formed at an arc-shaped distal end portion connecting the ridge lines 115b1 and 115b2 and portions of the ridge lines 115b1 and 115b2 on the both sides.

An electric cable 116 connected to a power source device (not shown) is coupled to a proximal end portion of the transducer 113. The grasping portion 111 includes a movable grasping portion 111a and a fixed grasping portion 111b. The movable grasping portion 111a is fixed with the transducer 113 and the ultrasonic probe 112. The fixed grasping portion 111b is fixed with the sheath portion 110. An elastic member 103 for biasing the movable grasping portion 111a in a direction in which the movable grasping portion 111a is separated from the fixed grasping portion 111b is disposed between the movable grasping portion 111a and the fixed grasping portion 111b. The probe distal end portion 115 is maintained in a state that it has been retreated inside the sheath portion 110 by spring force of the elastic member 103.

The movable grasping portion 111a is relatively moved so as to approach the fixed grasping portion 111b against the elastic force of the elastic member 103. Thereby, the probe distal end section 115 is put in a position relationship where the probe distal end section 115 slightly projects from the distal end portion of the sheath portion 110.

Since the taper face 108 has been formed on the distal end portion of the sleeve 105, such a configuration can be obtained that, when the inner needle unit 102 is inserted and arranged in the cannula 104, a large step does not occur between an outer peripheral face of the sheath portion 110 and the sleeve 105.

As shown in FIG. 19 and FIG. 20, a distal end face 117 of the sheath portion 110 is formed in an approximately conical shape to include a probe insertion hole 118 at a center portion (an axial center portion) thereof. A plurality of, four in the embodiment, blade sections 117a project from a surface of the distal end face 117 of the sheath portion 110. The distal end face 117 of the sheath portion 110 and the blade portions 117a are made from transparent resin or the like.

One observation window 119 and three illumination windows 121 are provided in the distal end face 117. An image pickup unit 120 is disposed corresponding to the observation window 119. Light guide units 122 are disposed corresponding to three illumination windows 121, respectively.

The image pickup unit 120 includes an observation window glass lens 120a provided at a distal end thereof, an observation optical system 120b comprising plural lenses provided at a proximal end thereof, a cover glass 120c provided at a proximal end of the observation optical system 120b, and an image pickup device 120d such as CCD or CMOS provided at a proximal end of the cover glass 120c.

The image pickup unit 120 further includes a board 120e with various circuits. The board 120e is connected with the image pickup device 120d. The board 120e is connected with a signal cable 120f. The signal cable 120f is connected to a video processor or a monitor (not shown) via the electric cable 116.

The light guide unit 122 comprises an illumination window lens 122a and an LED 122b serving as a light emitting device. The LED 122b is connected with an electric cable 122c. The electric cable 122c is connected to a power source device (not shown) via the electric cable 116.

Rubber members 123 are disposed on an outer peripheral face of the ultrasonic probe 112 at node positions of ultrasonic vibration. Thereby, an inner peripheral face of the probe insertion hole 118 of the sheath portion 110 and an outer peripheral face of the ultrasonic probe 112 are spaced from each other such that they do not contact with each other during ultrasonic vibration.

Next, an operation of the trocar 101 thus configured will be explained. The inner needle unit 102 and the cannula 104 are preliminarily set in an assembled state thereof at a use time of the trocar 101 according to the embodiment, as shown in FIG. 18.

Subsequently, an operator presses a distal end of the sheath portion 110 of the trocar 101 on a body cavity wall of a patient. In this state, the operator supplies power to the light guide unit 122. Thereby, the LED 122b emits light. In this state, a body cavity wall video image is picked up by the image pickup unit 120 of the observation window 119, and it is extracted on a monitor (not shown).

Thereafter, the operator relatively moves the movable grasping portion 111a to cause the same to approach the fixed grasping portion 111b against elastic force of the elastic member 103. Thereby, the probe distal end section 115 is caused to slightly project from the distal end portion of the sheath portion 110 (a position of the distal end portion of the ultrasonic transducer 113 shown by dotted lines in FIG. 18 and FIG. 20). In this state, the operator operates an output control device (not shown) to supply electric energy to the ultrasonic transducer 113. Thereby, ultrasonic vibration is generated at the ultrasonic transducer 113. The ultrasonic vibration from the transducer 113 is amplified by the horn 114 to be transmitted to the ultrasonic probe 112. At this time, a body cavity wall is punctured by the probe distal end portion 115 while the body cavity wall is being opened by the probe distal end portion 115.

Subsequently, the operator conducts further pushing-in operation of the distal end of the probe 112. Thereby, the distal end of the probe 112 is inserted deeply while an opened portion formed by the ultrasonic probe 112 is gradually expanded by tissue opening conducted by the distal end of the ultrasonic probe 112 and the blade portions 117a at the distal end face 117of the sheath portion 110. At this time, the operator can control output of ultrasonic wave while observing a puncture state of the ultrasonic probe 112 distal end from a musle layer of an abdominal wall into a peritoneum on a monitor screen.

With the above configuration, the following effect can be obtained. In the trocar 101 according to the embodiment, the ultrasonic probe 112 is disposed on the center axis of the inner needle unit 102, and the image pickup unit 120 and the light guide unit 122 are incorporated in the sheath portion 110 near the distal end portion of the ultrasonic probe 112. Thereby, the operator can puncture a body cavity wall based upon the ultrasonic wave output during puncture operation of the body cavity wall utilizing the trocar 101 while confirming a puncture state into the abdominal wall. Therefore, such a trouble that the distal end of the ultrasonic probe 112 is brought in contact with an internal organ goes away, so that the cannula 104 can be inserted into a body cavity further safely and smoothly as compared with the conventional art.

Incidentally, in FIG. 19, such a configuration can be adopted that two illumination windows 121 are provided and two observation windows 119 are provided at the distal end face 117 of the sheath portion 110, which allows binocular observation. In this case, the center portion of the distal end of the ultrasonic probe 112 can be observed further accurately.

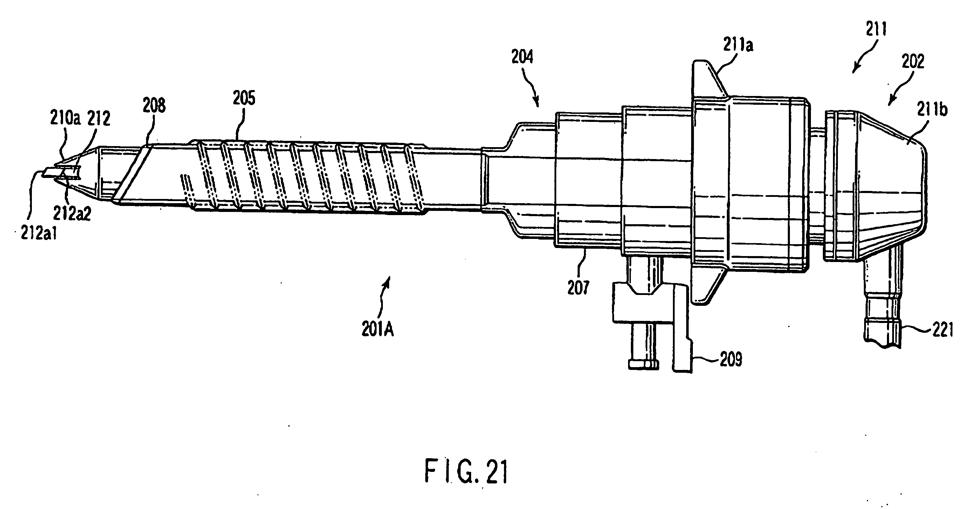

FIG. 21 to FIG. 28 show an embodiment of a trocar. FIG. 21 shows a whole configuration of a trocar 201A. The trocar 201A comprises a cannula 204 and an inner needle unit 202. The cannula 204 is a guide member for guiding an endoscope or a surgery instrument (not shown). The inner needle unit 202 can be inserted into and withdrawn from a through-hole of the cannula 204.

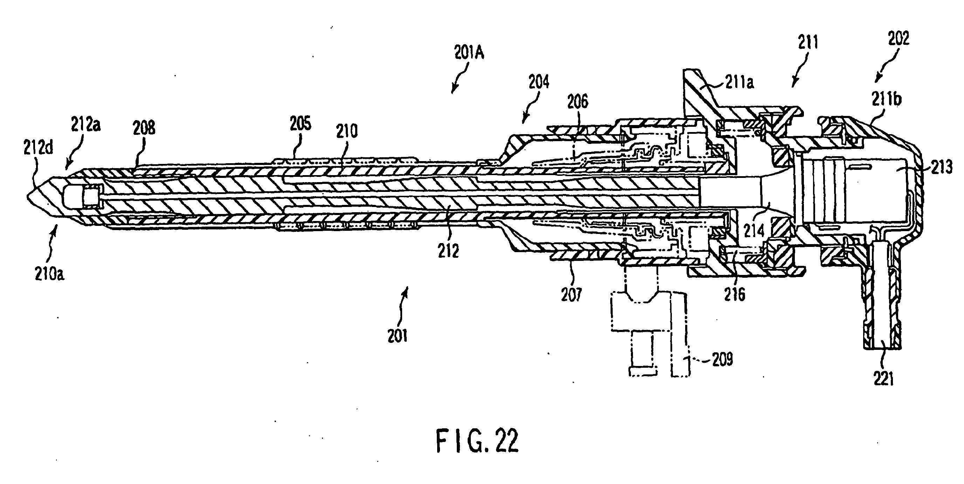

As shown in FIG. 22, the cannula 204 includes a sleeve 205 and a head member 207. The sleeve 205 is placed and fixed in a body cavity wall. The head member 207 is incorporated with a valve 206. The valve 206 holds an airtight state during insertion and withdrawal of an endoscope or a surgery instrument (not shown).

A taper face 208 tapered is formed on a distal end portion of the sleeve 205. An air-feeding mouth piece 209 for pneumoperitoneum gas is provided at the head member 207 in a projecting manner. The air-feeding mouth piece 209 communicates with the insertion hole of the sleeve 205.

The inner needle unit 202 comprises a sheath portion 210 and a grasping portion 211. The sheath portion 210 is a cover member that is inserted and arranged in the insertion hole of cannula 204. The grasping portion 211 is positioned at a proximal end portion of the sheath portion 210.

An ultrasonic transducer 213 and a horn 214 are provided in the sheath portion 210. The ultrasonic transducer 213 generates ultrasonic wave. The ultrasonic transducer 213 is provided at a proximal end portion of an ultrasonic probe 212. The horn 214 is provided at a distal end portion of the ultrasonic transducer 213. The horn 214 amplitudes ultrasonic vibration from the ultrasonic transducer 213 to transmit the same to the ultrasonic probe 212. A signal cable 221 connected to a power source device (not shown) is coupled to a proximal end portion of the ultrasonic transducer 213.

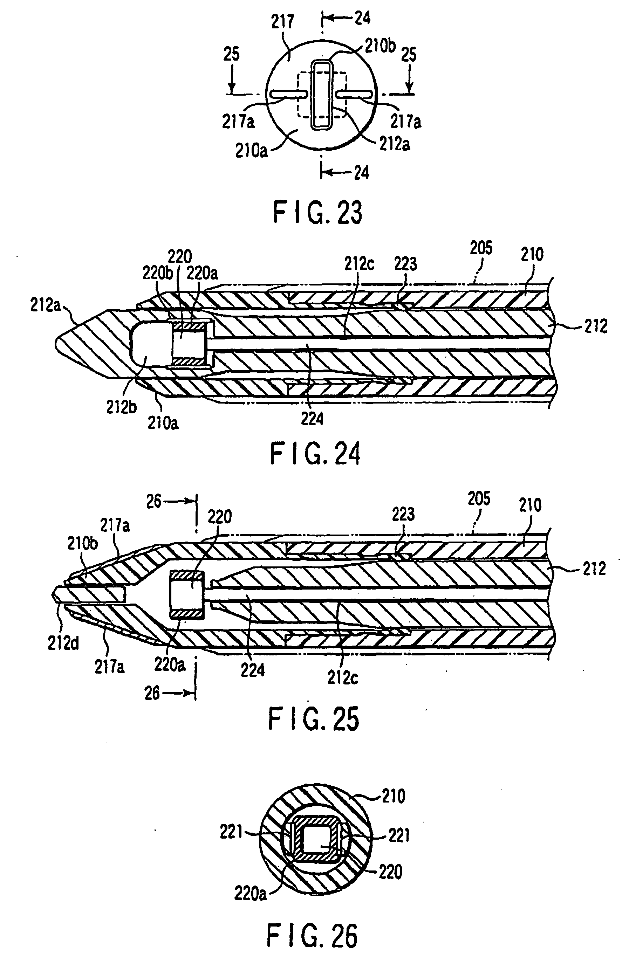

As shown in FIG. 23, a thin plate-shaped portion 212a is formed at a distal end portion of the ultrasonic probe 212. Thereby, a linear cutting opening can be obtained by ultrasonic-vibrating the thin plate-shaped portion 212a at the distal end portion of the ultrasonic probe 212 efficiently to bring the thin plate-shaped portion 212a in contact with a body tissue.

As shown in FIG. 24, a conical section 210a is formed at a distal end face 217 of the sheath portion 210. An elongate hole section 210b (see FIG. 23) extending vertically and serving as a probe insertion hole is formed at a central portion of the conical section 210a. The thin plate-shaped portion 212a of the ultrasonic probe 212 is disposed in the elongate hole portion 210b.

The grasping portion 211 comprises a movable grasping portion 211b and a fixed grasping portion 211a. The movable grasping portion 211b is fixed with the ultrasonic transducer 213 and the ultrasonic probe 212. The fixed grasping portion 211a is fixed with the sheath portion 210.

An elastic member 216 is interposed between the movable grasping portion 211b and the fixed grasping portion 211a. The elastic member 216 biases the movable grasping portion 211b in a direction in which the movable grasping portion 211b is separated from the fixed grasping portion 211a. Thereby, the ultrasonic probe 212 is held at a state that it has been retreated in a predetermined position inside the sheath portion 210 (shown by a retreating position 212a2 in FIG. 21).

The movable grasping portion 211b can be relatively moved to approach the fixed grasping portion 211a against an elastic force of the elastic member 216 of the fixed grasping portion 211a. Such a position relationship is set that, when the movable grasping portion 211b is relatively moved to approach the fixed grasping portion 211a, the thin plate-shaped portion 212a slightly projects from the conical section 210a (shown by a projecting position 212a1 in FIG. 21).

Since the taper face 208 is formed on the distal end portion of the sleeve 205, such a configuration can be obtained that, when the inner needle unit 202 is inserted and disposed in the cannula 204, a large step does not occur between the outer peripheral face of the probe 212 and the sleeve 205.

As shown in FIG. 23, a plurality of (two in the embodiment) blade sections 217a projects from a surface of the conical section 210a of the sheath portion 210. The distal end face 217 and the blade sections 217a are made from material such as transparent resin.

A hole section 212b is formed on a plate face of the thin plate-shaped portion 212a. An image pickup unit 220 is disposed in the hole portion 212b. Recesses are formed on a peripheral wall of the hole portion 212b of the thin plate-shaped portion 212a. Elastic members 220b for fixing the image pickup unit 220 are disposed in the recesses.

The image pickup unit 220 is fixed at a node portion of ultrasonic frequency via the elastic members 220b. Thereby, a configuration can be obtained that ultrasonic vibration of the probe 212 is not transmitted to the image pickup unit 220. A configuration can be achieved that the image pickup unit 220 is also moved according to advancing and retreating movements of the probe 212.

The image pickup unit 220 includes an objective observation optical system (not shown) and an image pickup device (not shown) such as CCD or CMOS. The objective observation optical system is disposed at a distal end of the image pickup unit 220. An image pickup device is provided at a proximal end portion of the objective observation optical system.

A light guide unit 220a is disposed on an outer periphery of the image pickup unit 220. The light guide unit 220a has at least one LED (not shown) serving as a light emitting device. The image pickup unit 220 and the light guide unit 220a are integrally assembled to each other. A circuit board (not shown) to which the image pickup device in the image pickup unit 220 and the LED in the light guide unit 220a are collectively connected and including various circuits is provided. The circuit board is connected with a signal cable 224.

A circular hole-shaped insertion hole 212c is formed at an axial center portion of the probe 212. An insertion hole (not shown) communicating with the insertion hole 212c of the probe 212 is formed at an axial center portion of the ultrasonic transducer 213. The signal cable 224 is inserted and disposed in the insertion hole 212c of the probe 212 and the insertion hole of the ultrasonic transducer 213. A proximal end portion of the signal cable 224 extending from the insertion hole of the ultrasonic transducer 213 is connected to the signal cable 221. Thereby, the image pickup device in the image pickup unit 220 and the LED in the light guide unit 220a are connected to a video processor and a monitor (not shown) via the signal cables 224 and 221.

Ring-like elastic members 223 are disposed on an outer peripheral face of the ultrasonic probe 212 at respective node positions of ultrasonic vibration. The outer peripheral face of the ultrasonic probe 212 and an inner peripheral face of the sheath 210 are held by the elastic members 223 so as to be spaced from each other. Thereby, the sheath portion 210 and the ultrasonic probe 212 are prevented from contacting with each other during ultrasonic vibration.

Next, an operation of the trocar 201A thus configured will be explained. The inner needle unit 202 and the cannula 204 are preliminarily set in an assembled state thereof at a use time of the trocar 201A according to the embodiment, as shown in FIG. 21. Subsequently, an operator presses the distal end portion of the sheath portion 210 of the trocar 201A on a body cavity wall of a patient. In this state, the operator supplies power to the light guide unit 220a. Thereby, the LED (not shown) emits light. In this state, an observation video image of the body cavity wall obtained from the image pickup unit 220 is displayed on a monitor (not shown).

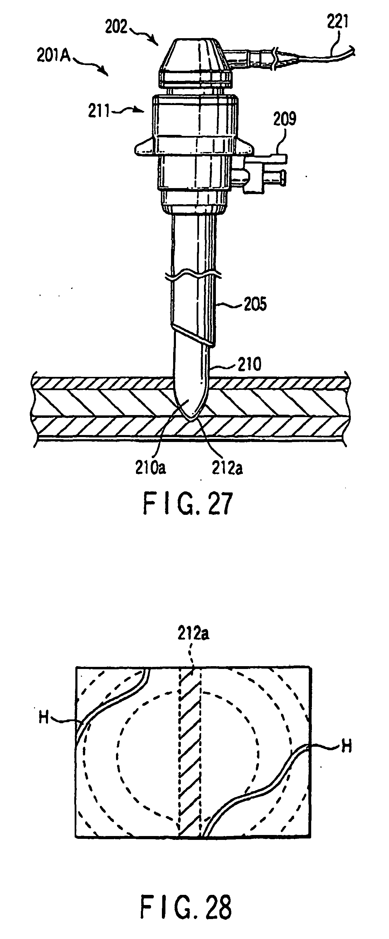

Thereafter, the operator relatively moves the movable grasping portion 211b so as to cause the same to approach the fixed grasping portion 211a against elastic force of the elastic member 216. Thereby, the thin plate-shaped portion 212a is caused to slightly project from the elongate hole portion 210b at the distal end of the sheath portion 210, as shown in FIG. 27. In this state, the operator operates an output control device (not shown) to supply electric energy to the ultrasonic transducer 213 and cause the ultrasonic transducer 213 to generate ultrasonic vibration.

After the ultrasonic vibration outputted from the ultrasonic transducer 213 is amplified by the horn 214, it is transmitted to the thin plate-shaped portion 212a via the ultrasonic probe 212. Thereby, the thin plate-shaped portion 212a projecting from the elongate hole portion 210b at the distal end portion of the sheath portion 210 is vibrated ultrasonically. Therefore, a linear opened portion is formed in a body tissue such as an abdominal wall by the thin plate-shaped portion 212a.

Then, the trocar 201A is inserted into a body tissue such as an abdominal wall while an opening portion obtained by the ultrasonic probe 212 is being gradually expanded by the plurality of blade portions 217a of the conical portion 210a of the sheath portion 210 and edge portions 212d of the thin plate-shaped portion 212a.

At this time, the operator can control output of ultrasonic wave while observing a puncture state of the distal end of the ultrasonic probe 212 from a muscle layer of an abdominal wall to a peritoneum by viewing a monitor image (see FIG. 28). At this time, as shown in FIG. 28, the thin plate-shaped portion 212a of the ultrasonic probe 212 is displayed on a central vertical axis on the monitor image. The operator can observe the puncture state of the distal end of the ultrasonic probe 212 while viewing a display portion of the thin plate-shaped portion 212a of the ultrasonic probe 212.

With the above configuration, the following effect can be achieved. In the embodiment, the thin plate-shape portion 212a is formed at the distal end portion of the ultrasonic probe 212. Thereby, for example, a volume and a mass of the thin plate-shaped portion 212a are considerably reduced as compared with those of an approximately conical distal end portion or the like. As a result, the thin plate-shaped portion 212a at the distal end portion of the ultrasonic probe 212 can be ultrasonically vibrated efficiently with small energy.

In the embodiment, only the edge portion 212d of the thin plate-shaped portion 212a is caused to project from the elongate hole portion 210b of the conical portion 210a of the sheath portion 210. Therefore, when the operator performs puncture operation in a state that he/she has pressed the distal end of the trocar 201A on a body tissue, he/she can insert the insertion portion of the cannula 204 into a target side smoothly while laterally expanding a linear opened portion formed in the body tissue by the edge portion 212d of the thin plate-shaped portion 212a. As a result, puncture utilizing the cannula 204 can be performed without opening a large hole in the body tissue unlike the case that the conical distal end portion is formed at a distal end of the ultrasonic probe 212.

In the embodiment, the image pickup unit 220 and the light guide unit 220a are fixed on a peripheral wall of the hole portion 212b of the thin plate-shaped portion 212a via the elastic members 220b disposed at the node position of the thin plate-shaped portion 212a of the ultrasonic probe 212. Thereby, the image pickup unit 220 and the light guide unit 220a are disposed so as to be hardly influenced by ultrasonic vibration from the thin plate-shaped portion 212a of the ultrasonic probe 212. As a result, the operator can perform puncture operation to a body cavity wall utilizing ultrasonic wave output during the puncture operation to the body cavity wall using the trocar 201A while confirming a puncture state into an abdominal wall. Accordingly, a trouble such that the distal end of the ultrasonic probe 212 is brought in contact with a body cavity organ is reduced so that the cannula 204 can be inserted into a body cavity wall further safely and smoothly as compared with the conventional art.

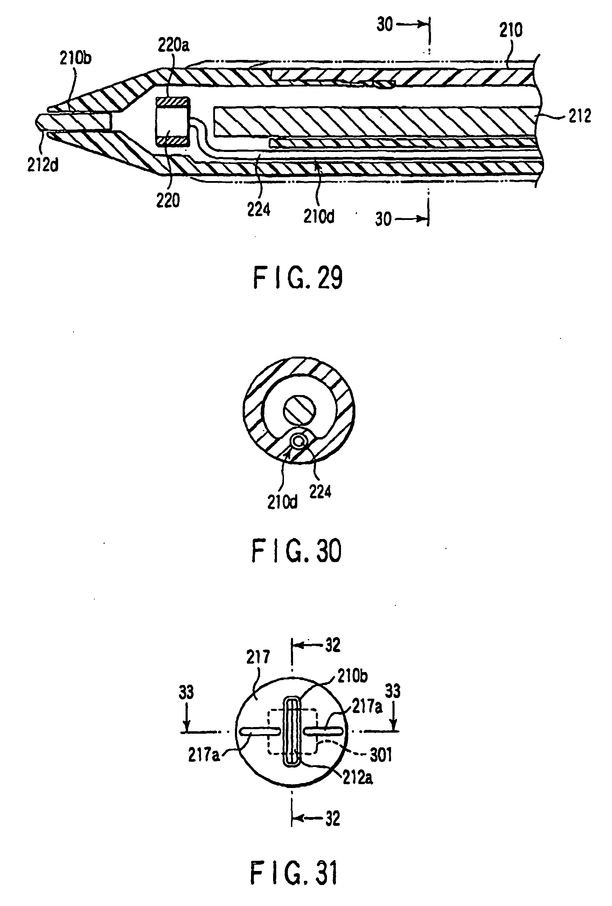

FIG. 29 and FIG. 30 show a modification of the trocar 201A according to the embodiment of FIGS. 21 to 28. The modification is obtained by modifying a shape of the sheath portion 210 shown in FIG. 25. That is, in the modification, as shown in FIG. 29 and FIG. 30, a cable insertion hole 210d is formed at an inner wall portion of a sheath portion 210. A signal cable 224 of a circuit board collectively connected with an image pickup device in the image pickup unit 220 and an LED in the light guide unit 220a is inserted and arranged in the cable insertion hole 210d of the sheath portion 210. In the modification, an effect similar to that in the embodiment of FIGS. 21 to 28 can also be obtained.

FIGS. 31 to 34 show a further embodiment of a trocar. The embodiment has a configuration obtained by modifying a configuration of the inner needle unit 202 in the trocar 201A according to the embodiment of FIG. 21 to FIG. 28 in the following manner. Here, only points modified from the trocar 201A according to the embodiment of FIGS. 21 to 28 will be explained.

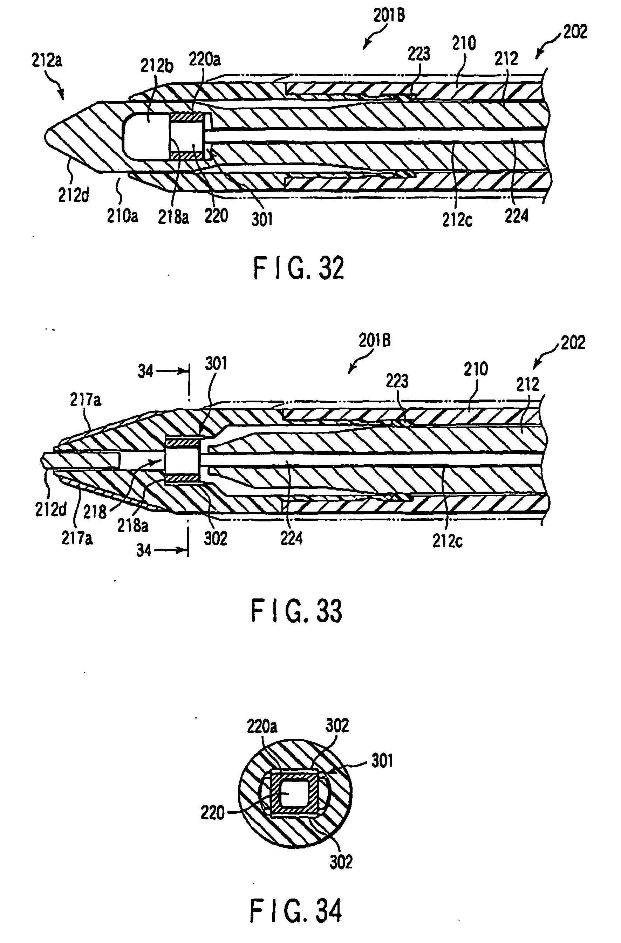

FIG. 31 to FIG. 34 show a configuration of a distal end of an inner needle unit 202. FIG. 31 is a front view showing a distal end portion of a trocar 201B according to this embodiment, viewed from a distal end of the trocar 201B, FIG. 32 is a sectional view showing an inner configuration of the distal end portion of the trocar 201B, taken along line 32-32 in FIG. 31, FIG. 33 is a sectional view showing the inner configuration of the distal end portion of the trocar 201B, taken along line 33-33 in FIG. 31, and FIG. 34 is a sectional view showing the inner configuration of the distal end portion of the trocar 201B, taken along line 34-34 in FIG. 33.

As shown in FIGS. 33 and 34, in an inner needle unit 202 according to the embodiment, an accommodating recessed portion 218 formed in a recessed shape is formed at a central position inside a conical portion 210a of the sheath portion 210. An assembling member 301 of an image pickup unit 220 and a light guide unit 220a is received in the accommodating recessed portion 218 so as to be inserted. The assembling member 301 of the image pickup unit 220 and the light guide unit 220a is caused to abut on a flat face 218a at a distal end of the accommodating recessed portion 218. In this state, the assembling member 301 is fixed and disposed in the accommodating recessed portion 218 in the sheath portion 210 via elastic members 302.

The image pickup unit 220 according to the embodiment is held so as to maintain a distance where, when a probe 212 is moved forward or rearward according to advancing or retreating of the movable grasping portion 211b, a hole portion 212b of a thin plate-shaped portion 212a and the image pickup unit 220 do not interfere with each other.

An ultrasonic transducer 213 (see FIG. 22) according to the embodiment is connected with a lead wire for ultrasonic vibration and a lead wire for high-frequency current. The signal wire for ultrasonic vibration and the signal wire for high-frequency current are inserted in a signal cable 221, respectively. A lead wire for ultrasonic vibration of the ultrasonic transducer 213 is connected to the signal wire for ultrasonic vibration of the signal cable 221. Similarly, a lead wire for high-frequency current of the ultrasonic transducer 213 is connected to the signal wire for high-frequency current of the signal cable 221. Accordingly, the lead wire for ultrasonic vibration and the lead wire for high-frequency current are disposed and connected to the signal cable, separately.

During driving of the trocar 201B according to the embodiment, ultrasonic vibration from the ultrasonic transducer 213 is transmitted to the probe 212 in the inner needle unit 202 so that ultrasonic wave procedure is conducted. High-frequency current is caused to flow in the probe 212 according to needs, so that high-frequency procedure is also conducted.

Next, an operation of the distal end portion of the trocar 2018 thus configured will be explained. In the embodiment, power is supplied to the light guide unit 220a. Thereby, the LED (not shown) emits light. In this state, an observation image of a body cavity wall obtained by the image pickup unit 220 is displayed on a monitor (not shown).

Thereafter, an operator relatively moves the movable grasping portion 211b to approach the fixed grasping portion 211a against elastic force of the elastic member 216. Thereby, as shown in FIG. 27, the operator causes the thin plate-shaped portion 212a to slightly project from the elongate hole portion 210b at the distal end of the sheath portion 210. In this state, the operator operates an output control device (not shown) to supply electric energy to the ultrasonic transducer 213, thereby generating ultrasonic vibration. At this time, ultrasonic vibration from the ultrasonic transducer 213 is transmitted to the probe 212 in the inner needle unit 202. Thereby, the thin plate-shaped portion 212a projecting from the elongate hole portion 210b at the distal end portion of the sheath portion 210 vibrates ultrasonically. Therefore, a linearly opened portion is formed in a body tissue such as an abdominal wall by the thin plate-shaped portion 212a.

In the embodiment, high-frequency current is caused to flow in the probe 212 according to needs. Thereby, a high-frequency procedure is conducted by the thin plate-shaped portion 212a projecting from the elongate hole portion 210b at the distal end portion of the sheath portion 210.

Then, the trocar 201B is inserted into a body tissue such as an abdominal wall by the plurality of blade portions 217a of the conical section 210a of the sheath portion 210 and edge portions 212d of the thin plate-shaped portion 212a while an opening portion is being gradually expanded by the ultrasonic probe 212.

At this time, the operator can controls output of ultrasonic wave while observing a puncture state of the distal end of the ultrasonic probe 212 from a muscle layer of an abdominal wall to a peritoneum by viewing a monitor image (see FIG. 28). At this time, as shown in FIG. 28, the thin plate-shaped portion 212a of the ultrasonic probe 212 is displayed on a central vertical axis on the monitor image. The operator can observe the puncture state of the distal end of the ultrasonic probe 212 while viewing a display portion of the thin plate-shaped portion 212a of the ultrasonic probe 212.

With the above configuration, the following effect can be achieved. In the embodiment, the image pickup unit 220 and the light guide unit 220a are fixed in the accommodating recessed portion 218 in the sheath 201 via the elastic members 302. Thereby, disposition can be made such that, when electric energy is supplied to the ultrasonic transducer 213, the image pickup unit 220 and the light guide unit 220 are hardly influenced by noises generated at the probe 212. Therefore, the operator outputs ultrasonic wave during a puncture operation to a body cavity wall conducted by the trocar 201B while confirming a puncture state to an abdominal wall. As a result, such a trouble that the distal end of the ultrasonic probe 112 is brought in contact with an organ in a body cavity is reduced, so that the cannula can be inserted into a body cavity wall further safely and smoothly as compared with the conventional art.

In the configuration, the image pickup unit 220 is not directly attached to the probe 212. Therefore, since high-frequency current can be caused to flow in the probe 212, puncture into a body cavity wall utilizing high-frequency current can be made possible. Here, for example, by viewing a monitor image during ultrasonic wave procedure (see FIG. 28), even if bleeding from a body cavity wall is observed, hemostasis can be conducted rapidly by causing high-frequency clotting current to flow in the probe 212. Therefore, safely can be further elevated.

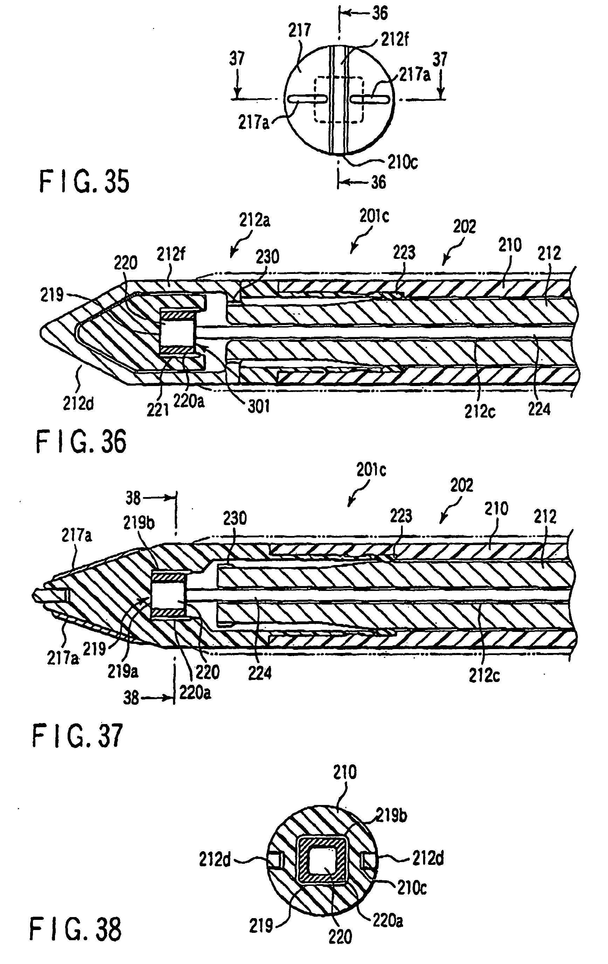

FIGS. 35 to 38 show a further embodiment of a trocar. The embodiment configuration obtained by modifying a configuration of the inner needle unit 202 in the trocar 201B according to the embodiment of FIG. 31 to FIG. 34 in the following manner. Here, only a modified point from the trocar 201B according to the embodiment of FIGS. 31 to 34 will be explained.

FIGS. 35 to 38 show a configuration of a distal end of the inner needle unit 202. FIG. 35 is a front view showing a distal end portion of a trocar 201C according to the embodiment, FIG. 36 is a sectional view showing an inner configuration of the distal end portion of the trocar 201C, taken along line 36-36 in FIG. 35, FIG. 37 is a sectional view showing the inner configuration of the distal end portion of the trocar 201C, taken along line 37-37 in FIG. 35, and FIG. 38 is a sectional view showing the inner configuration of the distal end portion of the trocar 201C, taken along line 38-38 in FIG. 37.

As shown in FIG. 35, a slit-shaped groove portion 210c for dividing a conical shape into two pieces is formed at a central portion on a conical portion 210a at a distal end of a sheath portion 210 of the inner needle unit 202. A thin plate-shaped portion 212f attachably and detachably accommodated along the groove portion 210c of the conical portion 210a is formed at a distal end portion of an ultrasonic probe 212.

The ultrasonic probe 212 is held in a submerged state that the thin plate-shaped portion 212f has been submerged in the groove portion 210c of the conical portion 210a of the sheath portion 210 by elastic force of the elastic member 216 positioned between the movable grasping portion 211b of the fixed grasping portion 211a of the grasping portion 211 like the ninth embodiment.

Such a position relationship is set that, when the movable grasping portion 211b is relatively moved to approach the fixed grasping portion 211a, the thin plate-shaped portion 212f slightly projects from the groove portion 210c of the conical section 210a of the sheath portion 210.

Incidentally, as shown in FIG. 36, the thin plate-shaped portion 212f of the ultrasonic probe 212 may be provided as a separate member from the probe 212. In this case, the probe 212 and the thin plate-shaped portion 212f separated therefrom may be joined to each other via a fixing member 230.

As shown in FIGS. 36 to 38, in the inner needle unit 202 according to the embodiment, an accommodating recessed portion 219 with a recessed shaped is formed at a central portion of the conical portion 210a of the sheath portion 210. As shown in FIG. 37, the accommodating recessed portion 219 of the sheath portion 210 is closed at its distal end portion. An assembling member 301 of the image pickup unit 220 and the light guide unit 220a is accommodated in the accommodated recessed portion 219 so as to be inserted.

The assembling member 301 of the image pickup unit 220 and the light guide unit 220a is caused to abut on a flat face 219a at a distal end of the accommodating recessed portion 219. The assembling member 301 is fixed and arranged in the accommodating recessed portion 219 of the sheath portion 210 via the elastic members 219b in a state where a gap between the flat face 219a at the distal end of the accommodating recessed portion 219 and a front face of the image pickup unit 220 has been removed.

The image pickup unit 220 is held so as to maintain a distance where, when a probe 212 is moved forward or rearward according to advancing or retreating of the movable grasping portion 211b, the probe 212 and the image pickup unit 220 do not interfere with each other.

Next, an operation of the distal end portion of the trocar 201C thus configured will be explained. In the embodiment, power is supplied to the light guide unit 220a. Thereby, the LED (not shown) emits light. In this state, an observation image of a body cavity wall obtained by the image pickup unit 220 is displayed on a monitor (not shown).

Thereafter, an operator relatively moves the movable grasping portion 211b to approach the fixed grasping portion 211a against elastic force of the elastic member 216 of the grasping portion 211. Thereby, the operator causes the thin plate-shaped portion 212f to slightly project from the groove portion 210c of the conical portion 210a of the sheath portion 210. In this state, the operator operates an output control device (not shown) to supply electric energy to the ultrasonic transducer 213, thereby generating ultrasonic vibration. At this time, ultrasonic vibration from the ultrasonic transducer 213 is transmitted to the probe 212 in the inner needle unit 202. Thereby, the thin plate-shaped portion 212f projecting from the groove portion 210c of the conical portion 210a of the sheath portion 210 vibrates ultrasonically. Therefore, a linearly opened portion is formed in a body tissue such as an abdominal wall by the thin plate-shaped portion 212f.

In the embodiment, high-frequency current is caused to flow in the probe 212 according to needs. Thereby, a high-frequency procedure is conducted by the thin plate-shaped portion 212f projecting from the groove portion 210c of the conical portion 210a of the sheath portion 210.

Subsequently, the trocar 201B is inserted into a body tissue such as an abdominal wall while an opened portion obtained by the ultrasonic probe 212 is being gradually expanded by the plurality of blade portions 217a of the conical portion 210a of the sheath portion 210 and edge portions 212d of the thin plate-shaped portion 212a.

At this time, the operator can control output of ultrasonic wave while observing a puncture state of the distal end of the ultrasonic probe 212 from a muscle layer of an abdominal wall to a peritoneum by viewing a monitor image (see FIG. 28). At this time, the thin plate-shaped portion 212f of the ultrasonic probe 212 is displayed on a central vertical axis on the monitor image. The operator can observe the puncture state of the distal end of the ultrasonic probe 212 while viewing a display portion of the thin plate-shaped portion 212f of the ultrasonic probe 212.

With the above configuration, the following effect can be achieved. In the embodiment, the image pickup unit 220 and the light guide unit 220a are fixed in the accommodating recessed portion 219 of the sheath portion 210 whose distal end has been closed via the elastic members 219b. Thereby, disposition can be made such that, when electric energy is supplied to the ultrasonic transducer 213, the image pickup unit 220 and the light guide unit 220a are hardly influenced by noises generated at the probe 212.

In the embodiment, the distal end portion of the accommodating recessed portion 219 in the sheath portion 210 is closed. Thereby, dusts, mist generated at a puncture time, or the like is prevented from adhering on a distal end lens face (not shown) of the image pickup unit 220.

The operator can outputs ultrasonic wave during a puncture operation into a body cavity wall using the trocar 201C while confirming a puncture state into an abdominal wall on a monitor screen (see FIG. 28). Thereby, a trouble such that the distal end of the ultrasonic probe 212 is brought in contact with a body cavity organ is reduced so that the cannula 204 can be inserted into a body cavity wall further safely and smoothly as compared with the conventional art.

In the configuration, the image pickup unit 220 is not directly attached to the probe 212. Therefore, since high-frequency current can be caused to flow in the probe 212, puncture into a body cavity wall utilizing high-frequency current can be made possible. Here, for example, by viewing a monitor image during ultrasonic wave procedure (see FIG. 28), even if bleeding from a body cavity wall is observed, hemostasis can be conducted rapidly by causing high-frequency clotting current to flow in the probe 212. Therefore, safely can be further elevated.

Other technical items featuring the present invention will be pointed out as supplemental descriptions.

### NOTE

(Additional Item 1) In an inner needle unit for a trocar including optical observation unit, ultrasonic wave energy applying unit or electric energy applying means for assisting body wall puncture is provided on an outer peripheral face of the inner needle unit.

(Additional Item 2) In the additional item 1, a center axis of the ultrasonic wave energy applying unit or the electric energy applying means in a longitudinal direction thereof is disposed on the same axis as a center axis of the inner needle unit in a longitudinal direction thereof.

(Additional Item 3) In the additional item 2, the ultrasonic wave energy applying unit is a tubular probe vibrating ultrasonically and a supporting member holding a distance between the inner needle unit and the probe is provided at a node position of ultrasonic vibration.

(Additional Item 4) In the additional item 3, the supporting member is an elastic member and is fixed on an outer peripheral face of the inner needle unit so as to be released against frictional resistance with the inner needle unit at insertion and withdrawal times.

(Additional Item 5) In the additional item 3, a slipping protective member with low frictional resistance is provided near a distal end of the inner needle unit.

(Additional Item 6) In the additional item 2, the electric energy applying unit is a tubular metal member whose inner face includes an insulating skin layer for maintaining insulation to the inner needle unit, and an insulating skin layer is provided on an outer peripheral face of the tubular metal member.

(Additional Item 7) In the additional item 6, the tubular metal member is obtained by disposing a plurality of tubular metal members with different inner diameters and outer diameters on the same axis and providing an insulating layer between the respective tubular metal members.

(Additional Item 8) In the additional item 2, the optical observation unit is an image pickup unit including an image pickup device and a circuit attached thereto, and the optical observation unit is provided at a position slightly deviated from a center major axis of the inner needle unit.

(Additional Item 9) In the additional item 8, illumination unit is provided near a distal end of the inner needle unit in parallel to an image pickup device unit.

(Additional Item 10) In an inner needle unit for a trocar including optical observation unit, ultrasonic wave energy applying unit or electric energy applying means for assisting body wall puncture is provided on an outer peripheral face of the inner needle unit and second optical observing means is provided outside the energy applying means.

(Additional Item 11) In the additional item 10, an image obtained by the second optical observing means is composed in an image processing system and is observed on the same screen.

(Additional Item 12) In the additional item 11, the second optical observing means is formed in an approximately annular shape.

(Additional Item 13) In a trocar comprising an inner needle that is inserted into a body cavity wall in a puncture manner and a cannula that is inserted and placed in the body cavity wall, the inner needle comprises a puncture needle that is transmitted with vibration energy or electric energy to be punctured in the abdominal cavity wall and a sheath that allows insertion of the puncture needle on a center axis thereof and is incorporated with observation optical means for displaying an observation image obtained by an image pickup device arranged around a distal end portion of an insertion portion of the puncture needle and illuminating means for emitting illumination light for illuminating a subject.

(Additional Item 14) The trocar according to the additional item 13 comprising an operation portion that allows projection and withdrawal of the distal end portion of the puncture needle to the distal end of the sheath.

(Additional Item 15) A trocar comprising an inner needle that is inserted into a body cavity wall in a puncture manner and a cannula that is inserted and placed in the body cavity wall, wherein the inner needle comprises a puncture needle that is transmitted with vibration energy or electric energy to puncture the body cavity wall, a sheath that allows insertion of the puncture needle on a center axis thereof, and objective observation optical means and illuminating means for emitting illumination light for illuminating a subject disposed at a distal end portion of the puncture needle.

(Additional Item 16) The trocar according to the additional item 15, wherein the objective observation optical means at the distal end portion of the puncture needle includes an image pickup device.

(Additional Item 17) The trocar according to the additional item 15, wherein the distal end portion of the puncture needle is formed in a thin plate shape.

(Additional Item 18) The trocar according to the additional item 15, wherein the observation optical means and illuminating means are attached on a center asix of the distal end portion of the puncture needle.

(Additional Item 19) The trocar according to the additional item 15, wherein a distal end face of the sheath is formed in a conical shape to include a slit portion serving as an insertion hole for the puncture needle, and a surface thereof includes a shape where a plurality of blade portions projects.

(Additional Item 20) The trocar according to the additional item 18, wherein the distal end portion of the puncture needle is attached with the observation optical means and the illuminating means via an elastic member at a node position of ultrasonic vibration.

(Additional Item 21) The trocar according to the additional item 15, wherein a signal cable for transmission in the observation optical means and the illumination means is inserted and arranged in a cylindrical insertion hole of the puncture needle.

(Additional Item 22) The trocar according to the additional item 15, wherein a signal cable for transmission in the observation optical means and the illumination means is inserted and arranged in an insertion hole of the sheath.

(Additional Item 23) A trocar comprising an inner needle that is inserted into a body cavity wall in a puncture manner and a cannula that is inserted and placed in the body cavity wall, wherein the inner needle comprises a puncture needle that is transmitted with vibration energy or electric energy to puncture the body cavity wall, a sheath that allows insertion of the puncture needle on a center axis thereof, and objective observation optical means and illuminating means for emitting illumination light for illuminating a subject disposed within a center at a distal end portion of the sheath.

(Additional Item 24) The trocar according to the additional item 23, wherein the objective observation optical means at the distal end portion of the puncture needle includes an image pickup device.

(Additional Item 25) The trocar according to the additional item 23, wherein the observation optical means and the illuminating means are attached inside the distal end of the sheath via an elastic member.

(Additional Item 26) The trocar according to the additional item 23, wherein an interior of the distal end of the sheath and a unit including the observation optical means and the illuminating means are attached to each other without any gap.

(Additional Item 27) The trocar according to the additional item 23, wherein a distal end face of the sheath is formed in a conical shape to include a slit portion serving as an mounting portion for the puncture needle, and a surface thereof includes a shape where a plurality of blade portions projects.

(Additional Item 28) The trocar according to the additional item 15 and the additional item 23, comprising an operation portion that allows projecting and withdrawing operations of the distal end portion of the puncture needle to the distal end of the sheath.

As effects of the additional items 15 and 22, an operator can puncture a body cavity wall utilizing ultrasonic wave energy or electric energy during a puncture operation to the body cavity wall while confirming a puncture state into an abdominal wall. Thereby, such a trouble that the distal end of the puncture needle is brought in contact with an internal organ is reduced, so that the cannula can be inserted into a body cavity further safely and smoothly as compared with the conventional art.

In addition to the above effects, the additional item 16 can reduce an opening formed in a tissue during puncturing or piercing. A distal end portion of an ultrasonic wave transmission member can be vibrated efficiently.

According to the additional item 18, puncture can be performed while an opened portion is being gradually expanded. Therefore, a low invasive trocar can be provided.

In addition to the above effects, according to the additional item 25, mist generated during puncturing of the like is prevented from adhering on a front face of a lens such as an image pickup device. Therefore, an image showing a puncture state in an abdominal wall and giving reduced stress to an operator can be provided.

## Claims

1. A trocar (1) comprising:
an inner needle unit (2) including optical observing means (4) and a viewing inner needle (42); and puncture-assisting means provided on an outer peripheral face of the inner needle unit (2), for assisting body wall puncture of the inner needle unit (2), wherein
the puncture-assisting means has at least ultrasonic energy applying means (81),
**characterized in that** the inner needle unit (2) has a slipping protecting member (40) with low friction coefficient configured to protect the viewing inner needle (42) from friction heat generation and abnormal noise generation due to ultrasonic vibration of the viewing inner needle unit (2), wherein the slipping protecting member (40) is provided at a distal end of a protective sheath (41) for protecting an insertion portion (42a) of the viewing inner needle (42).

2. The trocar (1) according to claim 1,
**characterized in that** the trocar (1) further comprises electric energy applying means (82), the ultrasonic energy applying means (81) and the electric energy applying means (82) each have a center axis line, and
the center axis line is disposed concentrically with a center axis line of the inner needle unit (2).

3. The trocar (1) according to claim 2,
**characterized in that** the ultrasonic energy applying means (81) has a tubular probe (11) vibrating in an ultrasonic manner, and
a supporting member holding a distance between the inner needle unit (2) and the probe (11) is provided at a node position of ultrasonic vibration of the probe (11).

4. The trocar (1) according to claim 3,
**characterized in that** the supporting member is an elastic member (16), and the elastic member (16) is fixed to an outer peripheral face of the inner needle unit (2) so as not to be detached from the outer peripheral face against frictional resistance generated at inserting and withdrawing times of the inner needle unit (2).

5. The trocar (1) according to claim 2,
**characterized in that** the electric energy applying means (82) has a tubular member (60) made from metal material, and
the tubular member (60) has an insulating skin layer (57) maintaining insulation against the inner needle unit (2) on an inner peripheral face thereof and has an insulating skin layer (51) on an outer peripheral face.

6. The trocar (1) according to claim 5,
**characterized in that** the tubular member is configured by arranging a plurality of tubular metal members (54, 55) having different inner diameters and outer diameters coaxially and providing an insulating layer (53) between the respective tubular metal members (54, 55).

7. The trocar (1) according to claim 2,
**characterized in that** the optical observing means is an image pickup device unit (38) including an image pickup device and a circuit attached thereto, and
the image pickup device unit (38) is provided at an eccentric position from the center axis line of the inner needle unit (2) near a distal end of the inner needle unit (2).

8. The trocar (1) according to claim 7,
**characterized in that** the inner needle unit (2) is provided near a distal end thereof with illuminating means (39) positioned in parallel with the image pickup unit (38).

9. The trocar (1) according to claim 1,
**characterized in that** the second optical observing means (46) is provided outside the ultrasonic energy applying means (81).

10. The trocar (1) according to claim 9,
**characterized by** further comprising an image pickup section (5A) that picks up an image obtained by the optical observing means (26),
a second image pickup section (46A) that picks up an image obtained by the second optical observing means (46), and
image processing means (6) that is connected to the image pickup section (5A) and the second image pickup section (46A), respectively, and synthesizes an image from the images pickup section (5A) and an image from the second image pickup section (46A) to display the images on the same screen.

11. The trocar (1) according to claim 10,
**characterized in that** the second optical observing means (46) is formed in an annular shape surrounding an periphery of the optical observing unit (26).

## Patentansprüche

1. Trokar (1) mit:
einer inneren Nadeleinheit (2), die ein optisches Beobachtungsmittel (4) und eine innere Betrachtungsnadel (42) aufweist; und einem Punktionshilfsmittel, das an einer Außenumfangsfläche der inneren Nadeleinheit (2) vorgesehen ist, um eine Punktion der Körperwandung durch die innere Nadeleinheit (2) zu unterstützen, wobei
das Punktionshilfsmittel mindestens ein Ultraschallenergie-Anwendungsmittel (81) aufweist,
**dadurch gekennzeichnet, dass** die innere Nadeleinheit (2) ein Gleitschutzelement (40) mit niedrigem Reibungskoeffizienten hat, das konfiguriert ist, um die innere Betrachtungsnadel (42) vor Erzeugung von Reibungswärme und vor anormaler Geräuschentwicklung infolge einer Ultraschallschwingung der inneren Betrachtungsnadel (42) zu schützen, wobei das Gleitschutzelement (40) an einem distalen Ende einer Schutzhülse (41) zum Schützen eines Einführabschnitts (42a) der inneren Betrachtungsnadel (42) vorgesehen ist.

2. Trokar (1) nach Anspruch 1,
**dadurch gekennzeichnet, dass** der Trokar (1) ferner ein Mittel zur Anwendung von elektrischer Energie (82) umfasst, wobei das Ultraschallenergie-Anwendungsmittel (81) und das Mittel zur Anwendung von elektrischer Energie (82) jeweils eine Mittelachslinie haben, und
die Mittelachslinie konzentrisch mit einer Mittelachslinie der inneren Nadeleinheit (2) angeordnet ist.

3. Trokar (1) nach Anspruch 2,
**dadurch gekennzeichnet, dass** das Ultraschallenergie-Anwendungsmittel (81) eine rohrförmige Sonde (11) aufweist, die ultraschallartig vibriert, und
ein Halterungselement, das einen Abstand zwischen der inneren Nadeleinheit (2) und der Sonde (11) hält, an einer Knotenstelle einer Ultraschallschwingung der Sonde (11) vorgesehen ist.

4. Trokar (1) nach Anspruch 3,
**dadurch gekennzeichnet, dass** das Halterungselement ein elastisches Element (16) ist, und das elastische Element (16) an einer Außenumfangsfläche der inneren Nadeleinheit (2) derart befestigt ist, dass es gegen Reibungswiderstand, der beim Einführen und Zurückziehen der inneren Nadeleinheit (2) erzeugt wird, nicht von der Außenumfangsfläche losgelöst wird.

5. Trokar (1) nach Anspruch 2,
**dadurch gekennzeichnet, dass** das Mittel zur Anwendung von elektrischer Energie (82) ein aus Metallmaterial gefertigtes, rohrförmiges Element (60) aufweist, und
das rohrförmige Element (60) eine isolierende Hautschicht (57) hat, die gegenüber der inneren Nadeleinheit (2) an seiner Innenumfangsfläche dauerhaft isoliert, und eine isolierende Hautschicht (57) an einer Außenumfangsfläche hat.

6. Trokar (1) nach Anspruch 5,
**dadurch gekennzeichnet, dass** das rohrförmige Element durch koaxiales Anordnen mehrerer rohrförmiger Metallelemente (54, 55) mit unterschiedlichen Innendurchmessern und Außendurchmessern und durch Bereitstellen einer Isolierschicht (53) zwischen den jeweiligen rohrförmigen Metallelementen (54, 55) konfiguriert ist.

7. Trokar (1) nach Anspruch 2,
**dadurch gekennzeichnet, dass** das optische Beobachtungsmittel eine Bildaufnahmegerät-Einheit (38) mit einem Bildaufnahmegerät und einer daran angebrachten Schaltung ist, und
die Bildaufnahmegerät-Einheit (38) an einer zur Mittelachslinie der inneren Nadeleinheit (2) exzentrischen Position nahe einem distalen Ende der inneren Nadeleinheit (2) vorgesehen ist.

8. Trokar (1) nach Anspruch 7,
**dadurch gekennzeichnet, dass** die innere Nadeleinheit (2) nahe einem distalen Ende derselben mit einem parallel zu der Bildaufnahmeeinheit (38) positionierten Beleuchtungsmittel (39) versehen ist.

9. Trokar (1) nach Anspruch 1,
**dadurch gekennzeichnet, dass** das zweite optische Beobachtungsmittel (46) außerhalb des Ultraschallenergie-Anwendungsmittels (81) vorgesehen ist.

10. Trokar (1) nach Anspruch 9,
**dadurch gekennzeichnet, dass** er ferner umfasst: einen Bildaufnahmeabschnitt (5A), der ein von dem optischen Beobachtungsmittel (26) erhaltenes Bild aufnimmt,
einen zweiten Bildaufnahmeabschnitt (46A), der ein von dem zweiten optischen Beobachtungsmittel (46) erhaltenes Bild aufnimmt, und
ein Bildverarbeitungsmittel (6), das jeweils mit dem Bildaufnahmeabschnitt (5A) und dem zweiten Bildaufnahmeabschnitt (46A) verbunden ist und ein Bild von dem Bildaufnahmeabschnitt (5A) und ein Bild von dem zweiten Bildaufnahmeabschnitt (46A) synthetisiert, um die Bilder auf demselben Bildschirm anzuzeigen.

11. Trokar (1) nach Anspruch 10,
**dadurch gekennzeichnet, dass** das zweite optische Beobachtungsmittel (46) in einer Ringform, welche einen Umfang der optischen Beobachtungseinheit (26) umgibt, ausgebildet ist.

## Revendications

1. Trocart (1), comprenant :
un module d'aiguille intérieure (2) incluant un moyen d'observation optique (4) et une aiguille intérieure de visualisation (42) ; et
un moyen d'assistance de ponction disposé sur une face périphérique extérieure du module d'aiguille intérieure (2), destiné à assister une perforation de paroi corporelle du module d'aiguille intérieure (2), dans lequel
le moyen d'assistance de ponction comporte au moins un moyen d'application d'énergie ultrasonore (81),
**caractérisé en ce que** le module d'aiguille intérieure (2) comporte un élément antiglissement (40) à bas coefficient de friction configuré pour protéger l'aiguille intérieure de visualisation (42) contre une production de chaleur de frottement et contre une production anormale de bruit dues à une vibration ultrasonore du module d'aiguille intérieure de visualisation (2), dans lequel l'élément anti-glissement (40) est disposé à une extrémité distale d'une gaine de protection (41) destinée à protéger une partie d'insertion (42a) de l'aiguille intérieure de visualisation (42).

2. Trocart (1) selon la revendication 1,
**caractérisé en ce que** le trocart (1) comprend en outre un moyen d'application d'énergie électrique (82), le moyen d'application d'énergie ultrasonore (81) et le moyen d'application d'énergie électrique (82) ont tous les deux un axe central, et
l'axe central est disposé concentriquement à un axe central du module d'aiguille intérieure (2).

3. Trocart (1) selon la revendication 2,
**caractérisé en ce que** le moyen d'application d'énergie ultrasonore (81) comporte une sonde tubulaire (11) vibrant de manière ultrasonore, et
un élément de support, maintenant une distance entre le module d'aiguille intérieure (2) et la sonde (11), est disposé à une position de noeud de vibration ultrasonore de la sonde (11).

4. Trocart (1) selon la revendication 3,
**caractérisé en ce que** l'élément de support est un élément élastique (16), et **en ce que** l'élément élastique (16) est fixé à une face périphérique extérieure du module d'aiguille intérieure (2) de façon à ne pas se désolidariser de la face périphérique extérieure lors d'une résistance au frottement produite lors d'une insertion et d'un retrait du module d'aiguille intérieure (2).

5. Trocart (1) selon la revendication 2,
**caractérisé en ce que** le moyen d'application d'énergie électrique (82) comporte un élément tubulaire (60) réalisé à partir d'un matériau métallique, et
l'élément tubulaire (60) comporte, sur sa face périphérique intérieure, une couche de peau isolante (57) maintenant une isolation par rapport au module d'aiguille intérieure (2) et comporte une couche de peau isolante (51) sur une face périphérique extérieure.

6. Trocart (1) selon la revendication 5,
**caractérisé en ce que** l'élément tubulaire est configuré par agencement d'une pluralité d'éléments métalliques tubulaires (54, 55) ayant des diamètres intérieurs et des diamètres extérieurs coaxiaux différents et établissant une couche isolante (53) entre les éléments métalliques tubulaires respectifs (54, 55).

7. Trocart (1) selon la revendication 2,
**caractérisé en ce que** le moyen d'observation optique est un module de dispositif de saisie d'image (38) incluant un dispositif de saisie d'image et un circuit qui lui est assujetti, et
le module de dispositif de saisie d'image (38) est disposé à une position excentrée de l'axe central du module d'aiguille intérieure (2) à proximité d'une extrémité distale du module d'aiguille intérieure (2).

8. Trocart (1) selon la revendication 7,
**caractérisé en ce que** le module d'aiguille intérieure (2) est pourvu, à proximité de son extrémité distale, d'un moyen d'illumination (39) placé parallèlement au module de saisie d'image (38).

9. Trocart (1) selon la revendication 1,
**caractérisé en ce que** le second moyen d'observation optique (46) est disposé à l'extérieur du moyen d'application d'énergie ultrasonore (81).

10. Trocart (1) selon la revendication 9,
**caractérisé par le fait qu'**il comprend en outre une section de saisie d'image (5A) qui saisit une image obtenue par le moyen d'observation optique (26),
une seconde section de saisie d'image (46A) qui saisit une image obtenue par le second moyen d'observation optique (46), et
un moyen de traitement d'images (6) qui est connecté respectivement à la section de saisie d'image (5A) et à la seconde section de saisie d'image (46A), respectivement et qui synthétise une image provenant de la section de saisie d'image (5A) et une image provenant de la seconde section de saisie d'image (46A) pour présenter les images sur le même écran.

11. Trocart (1) selon la revendication 10,
**caractérisé en ce que** le second moyen d'observation optique (46) est réalisé avec une forme annulaire entourant une périphérie du module d'observation optique (26).
